# EUROPEAN PATENT APPLICATION

(11) **EP 4 353 749 A1**
(43) Date of publication of application: **17.04.2024**
(21) Application number: 22819498.1
(22) Date of filing: 07.06.2022
(51) Int. Cl.: C07K 16/40, C12N 15/13

(54) **ANTI-MASP-2 ANTIBODY AND USE THEREOF**

(30) Priority: 08.06.2021 CN 202110638145
(71) Applicant: Shanghai Jemincare Pharmaceuticals Co., Ltd., Shanghai 201203 (CN); Jiangxi Jemincare Group Co., Ltd., Nanchang, Jiangxi 330096 (CN)
(72) Inventor: LIU, Xiaowu, Shanghai 201203 (CN); CAO, Xiaodan, Shanghai 201203 (CN); SONG, Jianqiu, Shanghai 201203 (CN); WANG, Zongda, Shanghai 201203 (CN); LIU, Peipei, Shanghai 201203 (CN); ZHANG, Jianjian, Shanghai 201203 (CN); GU, Chunyin, Shanghai 201203 (CN); DENG, Sujun, Shanghai 201203 (CN); PAN, Zhongzong, Shanghai 201203 (CN); WANG, Xueping, Shanghai 201203 (CN)
(74) Representative: Elzaburu S.L.P.
(86) International application number: PCT/CN2022/097268
(87) International publication number: WO 2022/257900

(57) **Abstract**

An anti-MASP-2 antibody and use thereof. The anti-MASP-2 antibody specifically binds to a human MASP-2 protein at a *K_{D}* value of about 2E-09M or less, and the anti-MASP-2 antibody specifically binds to a cynomolgus monkey MASP-2 protein at a *K_{D}* value of about 2E-09M or less. Disclosed are an immunoconjugate containing the anti-MASP-2 antibody, a method for preparing the anti-MASP-2 antibody, and use of the anti-MASP-2 antibody.

## Description

### TECHNICAL FIELD

The present application relates to the field of biomedicine, and in particular, to an anti-MASP-2 antibody and use thereof.

### BACKGROUND

Immunoglobulin A nephropathy (IgAN), also known as a Berger disease, is a type of mesangial proliferative glomerulonephritis (GN) characterized by diffuse deposition of IgA within a renal mesangium. At present, for the treatment of IgA nephropathy, KIDGO guidelines recommend using of ACEi/ARB to reduce urinary protein and improve renal function. Meanwhile, immunofluorescence studies have shown that activation of local complement C3 in the glomerularwas related to poor prognosis, suggesting that IgA nephropathy was associated with the activation of complement system. The complement system includes three pathways: 1) a classical pathway (CP); 2) an alternative pathway (AP); and 3) a lectin pathway (LP). In the past, the medical community generally believed that this disease had good prognosis and would not harm health. However, as time passed, doctors gained a deeper understanding of IgA nephropathy and found that not all IgA nephropathy patients had such good prognosis, and a considerable number of IgA nephropathy patients developed uremia 10 to 20 years after onset. If the rapid progression of IgA nephropathy is not well controlled, a huge burden will brought to the family and society. However, currently there are relatively few "weapons" for treating IgA nephropathy. Although RAS blockers (such as sartan and prils) and hormones can control the conditions of IgA nephropathy patients, a considerable proportion of patients have poor responses to these drugs. Abnormal activation of complements leading to kidney damage is currently an important direction of basic research, and exacerbation of some IgA nephropathy is caused by abnormal activation of complements.

MASP-2 is an effector enzyme of the lectin signaling pathway in the complement system, and is one of the ideal targets for preventing abnormal activation of complements.

Therefore, it is urgent to develop a novel anti-MASP-2 antibody with high affinity and strong specificity against MASP-2.

### SUMMARY OF THE INVENTION

The present application provides an isolated antigen binding protein, having one or more of the following properties: 1) in Octet detection, specifically binding to a human MASP-2 protein with a *K_{D}* value of about 2E-09M or less; 2) in Octet detection, specifically binding to a cynomolgus monkey MASP-2 protein with a *K_{D}* value of about 2E-09M or less; and 3) capable of specifically blocking a lectin pathway of a human complement system without affecting a classical pathway and an alternative pathway of the complement.

In some embodiments, the isolated antigen binding protein comprises HCDR3, and the HCDR3 comprises an amino acid sequence shown in SEQ ID NO: 19.

In some embodiments, the isolated antigen binding protein comprises HCDR2, and the HCDR2 comprises an amino acid sequence shown in SEQ ID NO: 17.

In some embodiments, the isolated antigen binding protein comprises HCDR1, and the HCDR1 comprises an amino acid sequence shown in SEQ ID NO: 15.

In some embodiments, the isolated antigen binding protein comprises HCDR1, HCDR2, and HCDR3 of a heavy chain variable region VH shown in SEQ ID NO: 67.

In some embodiments, the isolated antigen binding protein comprisesHCDR1, HCDR2, and HCDR3 of a heavy chain variable region VH shown in SEQ ID NO: 13 and SEQ ID NO: 26.

In some embodiments, the isolated antigen binding protein includes a heavy chain variable region VH, the VH comprises HCDR1, HCDR2, and HCDR3, and the HCDR3 comprises an amino acid sequence shown in SEQ ID NO: 19; the HCDR2 comprises an amino acid sequence shown in SEQ ID NO: 17; and the HCDR1 comprises an amino acid sequence shown in SEQ ID NO: 15.

In some embodiments, the isolated antigen binding protein includes H-FR1, a C terminal of the H-FR1 is directly or indirectly connected to an N terminal of the HCDR1, and the H-FR1 comprisesan amino acid sequence shown in SEQ ID NO: 62.

In some embodiments, the H-FR1 comprisesan amino acid sequence shown in any of SEQ ID NO: 14 and SEQ ID NO: 27.

In some embodiments, the isolated antigen binding protein comprises H-FR2, the H-FR2 is located between the HCDR1 and theHCDR2, and the H-FR2 comprises an amino acid sequence shown in SEQ ID NO: 63.

In some embodiments, the H-FR2 comprises an amino acid sequence shown in any of SEQ ID NO: 16 and SEQ ID NO: 28.

In some embodiments, the isolated antigen binding protein comprises H-FR3, the H-FR3 is located between the HCDR2 and theHCDR3, and the H-FR3 comprises an amino acid sequence shown in SEQ ID NO: 64.

In some embodiments, the H-FR3 comprises an amino acid sequence shown in any of SEQ ID NO: 18 and SEQ ID NO: 29.

In some embodiments, the isolated antigen binding protein comprisesH-FR4, an N terminal of the H-FR4 is directly or indirectly connected to a C terminal of the HCDR3, and the H-FR4 comprises an amino acid sequence shown in SEQ ID NO: 65.

In some embodiments, the H-FR4 comprises an amino acid sequence shown in any of SEQ ID NO: 20 and SEQ ID NO: 30.

In some embodiments, the isolated antigen binding protein comprises H-FR1, H-FR2, H-FR3, and H-FR4, and the H-FR1 comprises an amino acid sequence shown in SEQ ID NO: 62; the H-FR2 comprises an amino acid sequence shown in SEQ ID NO: 63; the H-FR3 comprises an amino acid sequence shown in SEQ ID NO: 64; and the H-FR4 comprises an amino acid sequence shown in SEQ ID NO: 65.

In some embodiments, the H-FR1 comprises an amino acid sequence shown in any of SEQ ID NO: 14 and SEQ ID NO: 27; the H-FR2 comprises an amino acid sequence shown in any of SEQ ID NO: 16 and SEQ ID NO: 28; the H-FR3 comprises an amino acid sequence shown in any of SEQ ID NO: 18 and SEQ ID NO: 29; and the H-FR4 comprises an amino acid sequence shown in any of SEQ ID NO: 20 and SEQ ID NO: 30.

In some embodiments, the H-FR1, H-FR2, H-FR3, and H-FR4 in the isolated antigen binding protein comprise amino acid sequences selected from any of the following groups:
a) H-FR1: SEQ ID NO: 14, H-FR2: SEQ ID NO: 16, H-FR3: SEQ ID NO: 18, and H-FR4: SEQ ID NO: 20; and
b) H-FR1: SEQ ID NO: 27, H-FR2: SEQ ID NO: 28, H-FR3: SEQ ID NO: 29, and H-FR4: SEQ ID NO: 30.

In some embodiments, the isolated antigen binding protein comprises a heavy chain variable region VH, and the VH comprises an amino acid sequence shown in SEQ ID NO: 67.

In some embodiments, the isolated antigen binding protein comprises a heavy chain variable region VH, and the VH comprises an amino acid sequence shown in any of SEQ ID NO: 13 and SEQ ID NO: 26.

In some embodiments, the isolated antigen binding protein comprises LCDR3, and the LCDR3 comprises an amino acid sequence shown in SEQ ID NO: 11.

In some embodiments, the isolated antigen binding protein comprises LCDR2, and the LCDR2 comprises an amino acid sequence shown in SEQ ID NO: 9.

In some embodiments, the isolated antigen binding protein comprises LCDR1, and the LCDR1 comprises an amino acid sequence shown in SEQ ID NO: 7.

In some embodiments, the isolated antigen binding protein comprises LCDR1, LCDR2, and LCDR3 of a light chain variable region VL shown in SEQ ID NO: 66.

In some embodiments, the isolated antigen binding protein comprises LCDR1, LCDR2, and LCDR3 of a light chain variable region VL shown in any of SEQ ID NO: 5 and 21.

In some embodiments, the isolated antigen binding protein comprises a light chain variable region VL, the VL comprises LCDR1, LCDR2, and LCDR3, and the LCDR3 comprises an amino acid sequence shown in SEQ ID NO: 11; the LCDR2 comprises an amino acid sequence shown in SEQ ID NO: 9; and the LCDR1 comprises an amino acid sequence shown in SEQ ID NO: 7.

In some embodiments, the isolated antigen binding protein comprises L-FR1, a C terminal of the L-FR1 is directly or indirectly connected to an N terminal of the LCDR1, and the L-FR1 comprises an amino acid sequence shown in SEQ ID NO: 58.

In some embodiments, the L-FR1 contains an amino acid sequence shown in any of SEQ ID NO: 6 and SEQ ID NO: 22.

In some embodiments, the isolated antigen binding protein comprises L-FR2, the L-FR2 is located between the LCDR1 and the LCDR2, and the L-FR2 comprises an amino acid sequence shown in SEQ ID NO: 59.

In some embodiments, the L-FR2 comprises an amino acid sequence shown in any of SEQ ID NO: 8 and SEQ ID NO: 23.

In some embodiments, the isolated antigen binding protein comprises L-FR3, the L-FR3 is located between the LCDR2 and the LCDR3, and the L-FR3 comprises an amino acid sequence shown in SEQ ID NO: 60.

In some embodiments, the L-FR3 comprises an amino acid sequence shown in any of SEQ ID NO: 10 and SEQ ID NO: 24.

In some embodiments, the isolated antigen binding protein comprises L-FR4, an N terminal of the L-FR4 is directly or indirectly connected to a C terminal of the LCDR3, and the L-FR4 comprises an amino acid sequence shown in SEQ ID NO: 61.

In some embodiments, the L-FR4 in the isolated antigen binding protein comprises an amino acid sequence shown in any of SEQ ID NO: 12 and SEQ ID NO: 25.

In some embodiments, the isolated antigen binding protein comprises L-FR1, L-FR2, L-FR3, and L-FR4, and the L-FR1 comprises an amino acid sequence shown in SEQ ID NO: 58; the L-FR2 comprises an amino acid sequence shown in SEQ ID NO: 59; the L-FR3 comprises an amino acid sequence shown in SEQ ID NO: 60; and the L-FR4 comprises an amino acid sequence shown in SEQ ID NO: 61.

In some embodiments, the L-FR1 comprises an amino acid sequence shown in any of SEQ ID NO: 6 and SEQ ID NO: 22; the L-FR2 comprises an amino acid sequence shown in any of SEQ ID NO: 8 and SEQ ID NO: 23; the L-FR3 comprises an amino acid sequence shown in any of SEQ ID NO: 10 and SEQ ID NO: 24; and the L-FR4 comprises an amino acid sequence shown in any of SEQ ID NO: 12 and SEQ ID NO: 25.

In some embodiments, the L-FR1, L-FR2, L-FR3, and L-FR4 in the isolated antigen binding protein include amino acid sequences selected from any of the following groups:
a) L-FR1: SEQ ID NO: 6, L-FR2: SEQ ID NO: 8, L-FR3: SEQ ID NO: 10, and L-FR4: SEQ ID NO: 12; and
b) L-FR1: SEQ ID NO: 22, L-FR2: SEQ ID NO: 23, L-FR3: SEQ ID NO: 24, and L-FR4: SEQ ID NO: 25.

In some embodiments, the VL in the isolated antigen binding protein comprises an amino acid sequence shown in SEQ ID NO: 66.

In some embodiments, the VL comprises an amino acid sequence shown in any of SEQ ID NO: 5 and SEQ ID NO: 21.

In some embodiments, the VH and VL in the isolated antigen binding protein include amino acid sequences selected from any of the following groups:
a) VH: SEQ ID NO: 13 and VL: SEQ ID NO: 5; and
b) VH: SEQ ID NO: 26 and VL: SEQ ID NO: 21.

In some embodiments, the isolated antigen binding protein comprises HCDR3, and the HCDR3 comprises an amino acid sequence shown in SEQ ID NO: 45.

In some embodiments, the isolated antigen binding protein comprises HCDR2, and the HCDR2 comprises an amino acid sequence shown in SEQ ID NO: 43.

In some embodiments, the isolated antigen binding protein comprises HCDR1, and the HCDR1 comprises an amino acid sequence shown in SEQ ID NO: 41.

In some embodiments, the isolated antigen binding protein comprises HCDR1, HCDR2, and HCDR3 of a heavy chain variable region VH shown in SEQ ID NO: 77.

In some embodiments, the isolated antigen binding protein comprises HCDR1, HCDR2, and HCDR3 of a heavy chain variable region VH shown in any of SEQ ID NO: 39 and SEQ ID NO: 52.

In some embodiments, the isolated antigen binding protein comprises a heavy chain variable region VH, the VH comprises HCDR1, HCDR2, and HCDR3, and the HCDR3 comprises an amino acid sequence shown in SEQ ID NO: 45; the HCDR2 comprises an amino acid sequence shown in SEQ ID NO: 43; and the HCDR1 comprises an amino acid sequence shown in SEQ ID NO: 41.

In some embodiments, the isolated antigen binding protein comprises H-FR1, a C terminal of the H-FR1 is directly or indirectly connected to an N terminal of the HCDR1, and the H-FR1 comprises an amino acid sequence shown in SEQ ID NO: 72.

In some embodiments, the H-FR1 in the isolated antigen binding protein comprises an amino acid sequence shown in any of SEQ ID NO: 40 and SEQ ID NO: 53.

In some embodiments, the isolated antigen binding protein comprises H-FR2, the H-FR2 is located between the HCDR1 and the HCDR2, and the H-FR2 comprises an amino acid sequence shown in SEQ ID NO: 73.

In some embodiments, the H-FR2 in the isolated antigen binding protein comprises an amino acid sequence shown in any of SEQ ID NO: 42 and SEQ ID NO: 54.

In some embodiments, the isolated antigen binding protein comprises H-FR3, the H-FR3 is located between the HCDR2 and the HCDR3, and the H-FR3 comprises an amino acid sequence shown in SEQ ID NO: 74.

In some embodiments, the H-FR3 in the isolated antigen binding protein comprises an amino acid sequence shown in any of SEQ ID NO: 44 and SEQ ID NO: 55.

In some embodiments, the isolated antigen binding protein comprises H-FR4, an N terminal of the H-FR4 is directly or indirectly connected to a C terminal of the HCDR3, and the H-FR4 comprises an amino acid sequence shown in SEQ ID NO: 75.

In some embodiments, the isolated antigen binding protein comprises H-FR4, an N terminal of the H-FR4 is directly or indirectly connected to a C terminal of the HCDR3, and the H-FR4 comprises an amino acid sequence shown in any of SEQ ID NO: 46 and SEQ ID NO: 30.

In some embodiments, the isolated antigen binding protein comprises H-FR1, H-FR2, H-FR3, and H-FR4, and the H-FR1 comprises an amino acid sequence shown in SEQ ID NO: 72; the H-FR2 comprises an amino acid sequence shown in SEQ ID NO: 73; the H-FR3 comprises an amino acid sequence shown in SEQ ID NO: 74; and the H-FR4 comprises an amino acid sequence shown in SEQ ID NO: 75.

In some embodiments, the H-FR1 in the isolated antigen binding protein comprises an amino acid sequence shown in any of SEQ ID NO: 40 and SEQ ID NO: 53; the H-FR2 comprises an amino acid sequence shown in any of SEQ ID NO: 42 and SEQ ID NO: 54; the H-FR3 comprises an amino acid sequence shown in any of SEQ ID NO: 44 and SEQ ID NO: 55; and the H-FR4 comprises an amino acid sequence shown in any of SEQ ID NO: 46 and SEQ ID NO: 30.

In some embodiments, the H-FR1, H-FR2, H-FR3, and H-FR4 in the isolated antigen binding protein compriseamino acid sequences selected from any of the following groups:
a) H-FR1: SEQ ID NO: 40, H-FR2: SEQ ID NO: 42, H-FR3: SEQ ID NO: 44, and H-FR4: SEQ ID NO: 46; and
b) H-FR1: SEQ ID NO: 53, H-FR2: SEQ ID NO: 54, H-FR3: SEQ ID NO: 55, and H-FR4: SEQ ID NO: 30.

In some embodiments, the isolated antigen binding protein comprises a heavy chain variable region VH, and the VH comprises an amino acid sequence shown in SEQ ID NO: 77.

In some embodiments, the VH in the isolated antigen binding protein comprises an amino acid sequence shown in any of SEQ ID NO: 39 and SEQ ID NO: 52.

In some embodiments, the isolated antigen binding protein comprises LCDR3, and the LCDR3 comprises an amino acid sequence shown in SEQ ID NO: 37.

In some embodiments, the isolated antigen binding protein comprises LCDR2, and the LCDR2 comprises an amino acid sequence shown in SEQ ID NO: 35.

In some embodiments, the isolated antigen binding protein comprises LCDR1, and the LCDR1 comprises an amino acid sequence shown in SEQ ID NO: 33.

In some embodiments, the isolated antigen binding protein comprises LCDR1, LCDR2, and LCDR3 of a light chain variable region VL shown in SEQ ID NO: 76.

In some embodiments, the isolated antigen binding protein comprises HCDR1, HCDR2, and HCDR3 of a light chain variable region VL shown in any of SEQ ID NO: 31 and SEQ ID NO: 47.

In some embodiments, the isolated antigen binding protein comprises a light chain variable region VL, the VL comprises LCDR1, LCDR2, and LCDR3, and the LCDR3 comprises an amino acid sequence shown in SEQ ID NO: 37; the LCDR2 comprises an amino acid sequence shown in SEQ ID NO: 35; and the LCDR1 comprises an amino acid sequence shown in SEQ ID NO: 33.

In some embodiments, the isolated antigen binding protein comprises L-FR1, a C terminal of the L-FR1 is directly or indirectly connected to an N terminal of the LCDR1, and the L-FR1 comprises an amino acid sequence shown in SEQ ID NO: 68.

In some embodiments, the L-FR1 in the isolated antigen binding protein comprises an amino acid sequence shown in any of SEQ ID NO: 32 and SEQ ID NO: 48.

In some embodiments, the isolated antigen binding protein comprises L-FR2, the L-FR2 is located between the LCDR1 and the LCDR2, and the L-FR2 comprises an amino acid sequence shown in SEQ ID NO: 69.

In some embodiments, the L-FR2 in the isolated antigen binding protein comprises an amino acid sequence shown in any of SEQ ID NO: 34 and SEQ ID NO: 49.

In some embodiments, the isolated antigen binding protein comprises L-FR3, the L-FR3 is located between the LCDR2 and the LCDR3, and the L-FR3 comprises an amino acid sequence shown in SEQ ID NO: 70.

In some embodiments, the L-FR3 in the isolated antigen binding protein comprises an amino acid sequence shown in any of SEQ ID NO: 36 and SEQ ID NO: 50.

In some embodiments, the isolated antigen binding protein comprises L-FR4, an N terminal of the L-FR4 is directly or indirectly connected to a C terminal of the LCDR3, and the L-FR4 comprises an amino acid sequence shown in SEQ ID NO: 71.

In some embodiments, the L-FR4 in the isolated antigen binding protein comprises an amino acid sequence shown in any of SEQ ID NO: 38 and SEQ ID NO: 51.

In some embodiments, the isolated antigen binding protein comprises L-FR1, L-FR2, L-FR3, and L-FR4, and the L-FR1 comprises an amino acid sequence shown in SEQ ID NO: 68; the L-FR2 comprises an amino acid sequence shown in SEQ ID NO: 69; the L-FR3 comprises an amino acid sequence shown in SEQ ID NO: 70; and the L-FR4 comprises an amino acid sequence shown in SEQ ID NO: 71.

In some embodiments, the L-FR1 in the isolated antigen binding protein comprises an amino acid sequence shown in any of SEQ ID NO: 32 and SEQ ID NO: 48; the L-FR2 comprises an amino acid sequence shown in any of SEQ ID NO: 34 and SEQ ID NO: 49; the L-FR3 comprises an amino acid sequence shown in any of SEQ ID NO: 36 and SEQ ID NO: 50; and the L-FR4 comprises an amino acid sequence shown in any of SEQ ID NO: 38 and SEQ ID NO: 51.

In some embodiments, the L-FR1, L-FR2, L-FR3, and L-FR4 in the isolated antigen binding protein compriseamino acid sequences selected from any of the following groups:
a) L-FR1: SEQ ID NO: 32, L-FR2: SEQ ID NO: 34, L-FR3: SEQ ID NO: 36, and L-FR4: SEQ ID NO: 38; and
b) L-FR1: SEQ ID NO: 48, L-FR2: SEQ ID NO: 49, L-FR3: SEQ ID NO: 50, and L-FR4: SEQ ID NO: 51.

In some embodiments, the isolated antigen binding protein comprises VL, and the VL comprises an amino acid sequence shown in SEQ ID NO: 76.

In some embodiments, the VL in the isolated antigen binding protein comprises an amino acid sequence shown in any of SEQ ID NO: 31 and 47.

In some embodiments, the isolated antigen binding protein comprises a VH and a VL, and the VH and the VL include amino acid sequences selected from any of the following groups:
a) VH: SEQ ID NO: 39 and VL: SEQ ID NO: 31; and
b) VH: SEQ ID NO: 52 and VL: SEQ ID NO: 47.

In some embodiments, the isolated antigen binding protein comprises a heavy chain constant region, and the heavy chain constant region comprises a constant region derived from IgG or a constant region derived from IgY.

In some embodiments, the heavy chain constant region in the isolated antibody binding protein comprises a constant region derived from IgG.

In some embodiments, the heavy chain constant region in the isolated antigen binding protein comprises a constant region derived from IgG1, IgG2, IgG3, or IgG4.

In some embodiments, the heavy chain constant region in the isolated antigen binding protein comprises an amino acid sequence shown in SEQ ID NO: 56.

In some embodiments, the isolated antigen binding protein comprises a light chain constant region, and the light chain constant region comprises a constant region derived from Igκ or a constant region derived from Igλ.

In some embodiments, the light chain constant region in the isolated antigen binding protein comprises a constant region derived from human Igκ.

In some embodiments, the light chain constant region in the isolated antigen binding protein comprises an amino acid sequence shown in SEQ ID NO: 57.

In some embodiments, the isolated antigen binding protein comprises an antibody or an antigen binding fragment thereof.

In some embodiments, the antigen binding fragment in the isolated antigen binding protein is selected from the following group: Fab, Fab', F(ab)2, Fv fragment, F(ab')2, scFv, di-scFv, VHH, and/or dAb.

In some embodiments, the antibody in the isolated antigen binding protein is selected from the following group: a monoclonal antibody, a single chain antibody, a chimeric antibody, a humanized antibody, and a fully human antibody.

In another aspect, the present application provides one or more polypeptides, comprising the isolated antigen binding protein.

In another aspect, the present application provides one or more immunoconjugates, comprising the isolated antigen binding protein or the polypeptide.

In another aspect, the present application provides one or more isolated nucleic acid molecules, encoding the isolated antigen binding protein or the polypeptide.

In another aspect, the present application provides one or more vectors, comprising the isolated nucleic acid molecule.

In another aspect, the present application provides one or more cells, comprising the isolated antigen binding protein, the polypeptide, the immunoconjugate, the isolated nucleic acid molecule, and/or the vector.

In another aspect, the present application provides a method for preparing the isolated antigen binding protein or the polypeptide, the method including culturing the cell under the condition of expressing the isolated antigen binding protein or the polypeptide.

In another aspect, the present application provides one or more pharmaceutical compositions, comprising the isolated antigen binding protein, the polypeptide, the immunoconjugate, the isolated nucleic acid molecule, the vector, the cell, and/or pharmaceutically acceptable adjuvants and/or excipients.

In another aspect, the present application provides a method for detecting or measuring MASP-2, the method including use of the isolated antigen binding protein or the polypeptide.

In another aspect, the present application provides a detection kit for MASP-2, including the isolated antigen binding protein or the polypeptide.

In another aspect, the present application provides a use of the isolated antigen binding protein or the polypeptide in preparation of a kit for detecting the presence and/or content of MASP-2.

In another aspect, the present application provides a use of the isolated antigen binding protein, the polypeptide, the immunoconjugate, the isolated nucleic acid molecule, the vector, the cell, and/or the pharmaceutical composition in preparation of drugs for preventing and/or treating diseases or conditions.

In another aspect, the present application provides a use of the isolated antigen binding protein, the polypeptide, the immunoconjugate, the isolated nucleic acid molecule, the vector, the cell, and/or the pharmaceutical composition for preventing, alleviating, and/or treating diseases or conditions.

In another aspect, the present application provides a method for preventing and/or treating diseases or conditions, including administering, to subjects in need, an effective amount of the isolated antigen binding protein, the polypeptide, the immunoconjugate, the isolated nucleic acid molecule, the vector, and/or the cell.

Those skilled in the art can easily understand other aspects and advantages of the present application from the detailed description below. The detailed description below only shows and describes exemplary embodiments of the present application. As those skilled in the art will recognize, the content of the present application enables those skilled in the art to make modifications to the disclosed specific embodiments without departing from the spirit and scope of the invention referred to in the present application. Correspondingly, the descriptions in the accompanying drawings and specification of the present application are only illustrative and not restrictive.

### BRIEF DESCRIPTION OF THE DRAWINGS

Specific features of the invention referred to in the present application are shown in the appended claims. The characteristics and advantages of the invention referred to in the present application can be better understood by referring to the exemplary embodiments and accompanying drawings described in detail below. A brief explanation of the accompanying drawings is as follows:
FIG. 1 shows binding of an exemplary anti-MASP-2 antibody 50A6 described in the present application to human MASP-2.
FIG. 2 shows binding of an exemplary anti-MASP-2 antibody 47A1 described in the present application to human MASP-2.
FIG. 3A shows binding of an exemplary anti-MASP-2 antibody 50A6 described in the present application to cynomolgus monkey MASP-2.
FIG. 3B shows binding of an exemplary anti-MASP-2 antibody 50A6 described in the present application to mouse MASP-2.
FIG. 4A shows binding of an exemplary anti-MASP-2 antibody 47A1 described in the present application to cynomolgus monkey MASP-2.
FIG. 4B shows binding of an exemplary anti-MASP-2 antibody 47A1 described in the present application to mouse MASP-2.
FIG. 5 shows results of blocking a lectin pathway of a complement system by an exemplary anti-MASP-2 antibody described in the present application.
FIG. 6 shows results of blocking a lectin pathway of a complement system by an exemplary humanized anti-MASP-2 antibody described in the present application.
FIG. 7 shows pharmacokinetic (PK) results of an exemplary anti-MASP-2 antibody described in the present application in a humanized FcRn mouse model.

### DETAILED DESCRIPTION OF THE PREFERRED EMBODIMENTS

Implementations of the present application will be explained below by specific embodiments. Those familiar with the art can easily understand other advantages and effects of the present invention from the disclosure of this specification.

### Definition

In the present application, the term "isolated" usually refers to obtained by artificial means from a natural state. If an "isolated" substance or ingredient appears in the nature, a natural environment where the substance is located may be changed, or the substance is isolated from a natural environment, or the both occur. For example, a polynucleotide or polynucleotide that is not isolated naturally exists in a living animal, and the high-purity identical polynucleotide or polypeptide isolated from the natural state is referred to as isolated. The term "isolated" does not exclude artificial or synthetic substances or other impure substances that do not affect the activity of the substance.

In the present application, the term "antigen binding protein" usually refers to a polypeptide molecule that can specifically recognize and/or neutralize a specific antigen. For example, in the present application, the term "antigen binding protein" may include "antibody" or "antigen binding fragment". For example, the antibody may comprise an immunoglobulin composed of at least two heavy (H) chains and two light (L) chains interconnected by disulfide bonds, and may include any molecule containing its antigen binding portion. The term "antibody" may include monoclonal antibodies, antibody fragments or antibody derivatives, including but not limited to mouse derived antibodies, human antibodies (fully human antibodies), humanized antibodies, chimeric antibodies, single chain antibodies (such as scFv), and antibody fragments that bind to antigens (such as Fab, Fab', VHH, and (Fab)2 fragments). The term "antibody" may further include all recombinant forms of antibodies, such as antibodies expressed in prokaryotic cells, non-glycosylated antibodies, any antibody fragments binding to antigens as described herein and derivatives thereof. Each heavy chain may be composed of a heavy chain variable region (VH) and a heavy chain constant region. Each light chain may be composed of a light chain variable region (VL) and a light chain constant region. The VH and VL regions may be further distinguished as hypervariable regions referred to as complementary decision regions (CDR), which are scattered in more conservative regions referred to as framework regions (FR). Each VH and VL may be composed of three CDR regions and four FR regions, which may be arranged in the following order from an amino terminal to a carboxyl terminal: FR1, CDR1, FR2, CDR2, FR3, CDR3, and FR4. The variable regions of the heavy chain and the light chain include binding domains that interact with antigens (such as human MASP-2). The constant region of the antibody can mediate binding of the immunoglobulin to a host tissue or factor, and the host tissue or factor includes multiple kinds of cells (such as effector cells) of an immune system and a first component (Clq) of a classical complement system. Exact boundaries of the CDRs have been defined according to different systems. The system described by Kabat et al., Sequences of Proteins of Immunological Interest (National Institutes of Health, Bethesda, Md. (1987) and (1991)) not only provides a clear residue numbering system that can be applied to any variable region of an antigen binding fragment, but further provides exact residue boundaries that define CDRs. These CDRs may be referred to as Kabat CDRs. Chothia and his colleagues (Chothia and Lesk, J.Mol. Biol. 196: 901-917 (1987) and Chothia et al., Nature 342: 877-883 (1989)) found that sub-fractions in Kabat CDRs have almost identical peptide main chain conformations in spite of significant diversity at the amino acid sequence level. These sub-fractions are named L1, L2, and L3, or H1, H2, and H3, where "L" and "H" refer to light and heavy chain regions, respectively. These regions may be referred to as Chothia CDRs, and the Chothia CDRs have boundaries that overlap with Kabat CDRs. Other boundaries that overlap with Kabat CDRs and define CDRs have been described by Padlan (FASEB J.9: 133-139 (1995)) and MacCallum (J Mol Biol 262(5): 732-45 (1996)). In addition, the other CDR boundaries may not be strictly defined in accordance with one of the above systems, but still overlap with the Kabat CDRs. Although specific residues or groups of residues or even the entire CDRs do not significantly affect antigen binding predictions or experimental findings, they can be shortened or lengthened. In the present application, the CDR may be defined by the Chothia numbering system.

In the present application, the term "antigen-binding fragment" usually refers to one or more fragments in the antibody that perform a specific antigen binding function. The antigen binding function of the antibody may be achieved by a full length fragment of the antibody. The antigen binding function of the antibody may alternatively be achieved by a heavy chain including an Fv, ScFv, dsFv, Fab, Fab', or F(ab')2 fragment, or a light chain including an Fv, scFv, dsFv, Fab, Fab', or F(ab')2 fragment. (1) Fab fragment, usually a univalent fragment composed of VL, VH, CL, and CH domains; (2) F(ab')2 fragment, including a divalent fragment of two Fab fragments connected by a disulfide bond at a hinge region; (3) Fd fragment composed of VH and CH domains; (4) Fv fragment composed of VL and VH domains on a single arm of the antibody; (5) dAb fragment composed of a VH domain (Ward et al., (1989) Nature 341: 544-546); (6) an isolated complementary decision region (CDR); and (7) a combination of two or more isolated CDRs optionally connected by a linker. For example, the antigen-binding fragment may further include a monovalent single chain molecule Fv (scFv) formed by pairing VL and VH (see Bird et al. (1988) Science 242: 423-426; and Huston et al. (1988) Proc. Natl. Acad. Sci. 85: 5879-5883). For example, the antigen-binding fragment may further include a type of antibody VHH that lacks the light chain of the antibody and has only the heavy chain variable region (for example, see Kang Xiaozhen et al., Journal of Biotechnology, 2018, 34 (12): 1974-1984). The "antigen binding fraction" may further include an immunoglobulin fusion protein, which includes binding domains selected from the following: (1) a binding domain peptide fused with a polypeptide in an immunoglobulin hinge region; (2) an immunoglobulin heavy chain CH2 constant region fused with the hinge region; and (3) an immunoglobulin heavy chain CH3 constant region fused with the CH2 constant region.

In the present application, the term "monoclonal antibody" usually refers to a group of substantially homologous antibodies, namely, a group of antibodies that are identical except for possible mutants that exist in trace amounts and naturally occur. The monoclonal antibody is highly specific and directly targets a single antigenic site. For example, the monoclonal antibody may be prepared by a hybridoma technology or generated in bacterial, eukaryotic, or plant cells by a recombinant DNA method. The monoclonal antibody may alternatively be derived from a phage antibody library by technologies described in Clackson et al, Nature, 352:624-628 (1991) and Marks et al., Mol. Biol., 222:581-597 (1991).

In the present application, the term "chimeric antibody" usually refers to an antibody where a portion of each heavy or light chain amino acid sequence is homologous to a corresponding amino acid sequence in an antibody from a specific species, or belongs to a specific category, while the remaining segments of the chain are homologous to corresponding sequences in another species. For example, variable regions of both light and heavy chains come from variable regions of an antibody from one animal species (such as mice or rats), while constant portions are homologous to sequences of an antibody from another species (such as humans). For example, to obtain a chimeric antibody, non-human B cells or hybridoma cells may be used to generate variable regions, while constant regions combined with the variable regions come from humans. The variable region has the advantage of easy preparation, and its specificity is not affected by the source of the constant region combined with the variable region. Meanwhile, because the constant region of the chimeric antibody may come from humans, the likelihood of triggering an immune response by the chimeric antibody during injection is lower than that using a non-human antibody in the constant region.

In the present application, the term "humanized antibody" usually refers to a chimeric antibody that includes fewer sequences from a non-human immunoglobulin, thereby reducing the immunogenicity of heterologous antibodies introduced into humans and maintaining the complete antigen binding affinity and specificity of the antibody. For example, non-human binding domains can be humanized by technical means such as CDR transplants (Jones et al., Nature 321:522 (1986)) and variants thereof; "reshaping" (Verhoeyen, et al., 1988 Science 239:1534-1536; Riechmann, et al., 1988 Nature 332:323-337; Tempest, et al., Bio/Technol 1991 9:266-271), "hyperchimerization", (Queen, et al., 1989 Proc Natl Acad Sci USA 86:10029-10033; Co, et al., 1991 Proc Natl Acad Sci USA 88:2869-2873; Co, et al., 1992 J Immunol 148:1149-1154), and "veneering" (Mark, et al., "Derivation of thermally active humanized and veneered anti-CD18 antibiotics." In: Metcalf B W, Dalton B J, eds. Cellular adhesion: molecular definition to therapeutic potential. New York: Plenum Press, 1994: 291-312), and resurfacing (US patent US5639641). If domains of other regions, such as hinge regions and constant regions, also originate from non-human sources, these regions can also be humanized.

In the present application, the term "mouse derived antibody" usually refers to an antibody having a variable region framework and a CDR region derived from mouse germline immunoglobulin sequences. Moreover, if the antibody includes a constant region, the constant region is also derived from a mouse germline immunoglobulin sequence. The mouse derived antibody of the present application may include amino acid residues that are not encoded by mouse germline immunoglobulin sequences, for example, include mutants introduced by in vitro random mutations or point mutations or in vivo somatic mutations.

In the present application, the term "germline sequence" usually refers to a sequence of immunoglobulin DNA sequences that are not rearranged.

In the present application, the term "between" usually refers to direct or indirect connection between a C terminal of an amino acid fragment and an N terminal of a first amino acid fragment, and direct or indirect connection between an N terminal of the amino acid fragment and a C terminal of a second amino acid fragment. In the light chain, for example, the N terminal of the L-FR2 is directly or indirectly connected to the C terminal of the LCDR1, and the C terminal of the L-FR2 is directly or indirectly connected to the N terminal of the LCDR2. For another example, the N terminal of the L-FR3 is directly or indirectly connected to the C terminal of the LCDR2, and the C terminal of the L-FR3 is directly or indirectly connected to the N terminal of the LCDR3. In the heavy chain, for example, the N terminal of the H-FR2 is directly or indirectly connected to the C terminal of the HCDR1, and the C terminal of the H-FR2 is directly or indirectly connected to the N terminal of the HCDR2. For another example, the N terminal of the H-FR3 is directly or indirectly connected to the C terminal of the HCDR2, and the C terminal of the H-FR3 is directly or indirectly connected to the N terminal of the HCDR3.

In the present application, the terms "MASP-2 protein", "MASP-2", and "MASP-2 antigen" may be used interchangeably and include any functional active fragment, variant, and homolog of MASP-2 that is expressed naturally in cells or on cells transfected with MASP-2 genes. In the present application, MASP-2 may be human MASP-2, with Primary accession 000187 in UniProt/Swiss Prot. For example, MASP-2 may be a functional active fragment of human MASP-2. In the present application, MASP-2 may be cynomolgus monkey MASP-2 or a functional active fragment thereof. For example, the "functional active fragment" may include a fragment that retains the endogenous function of at least one natural protein (such as binding to the antigen binding protein described in the present application). For example, the "functional active fragment" may include a domain that binds to the antigen binding protein of the present application. In the present application, MASP-2 is a key regulatory factor in a lectin pathway of a complement system. Mannan-binding lectin (MBL) or fibronectin (FCN) in plasma directly recognizes saccharide structures with terminal saccharide residues such as mannose, N-acetylmannose, N-acetylglucosamine, and fucose on surfaces of many pathogenic microorganisms. An MBL-MASP complex binds to a saccharide structure on the surface of a pathogen to activate MASP-1 and MASP-2 independently. The activated MASP-2 exerts its SP activity to cleave C4, the resulting C4b fragment covalently binds to the surface of the pathogen, and the C4b fragment is also cleaved by MASP-2 through interaction with C2 to form C3 converting enzyme C4b2a, which then activates a classical pathway of a complement. The activated MASP1 can directly cleave C3 to produce C3b, which, under the action of protein factor D and protein factor P, forms a C3 converting enzyme C3bBb or C3bBbP, and produces a C5 converting enzyme C3bBb3b, to activate an alternative pathway of the complement.

In the present application, the term "IgA nephropathy" usually refers to primary glomerulopathy in a glomerular mesangial region. For example, the term "IgA nephropathy" may include primary glomerulopathy dominated by IgA or IgA deposition, with or without deposition of other immunoglobulins in the glomerular mesangial region. For example, pathological types of the "IgA nephropathy" may include focal segmental lesions, proliferative lesions in capillaries, mesangial proliferative lesions, crescent lesions, sclerotic lesions, and the like. Its clinical manifestation is recurrent gross hematuria or microscopic hematuria, accompanied by varying degrees of proteinuria. Some patients may experience severe hypertension or renal dysfunction.

In addition to the specific proteins and nucleotides mentioned herein, the present application may further include functional active fragments, derivatives, analogs, homologues and fragments thereof.

The term "functional active fragment" refers to one or more active polypeptides that have substantially a same amino acid sequence as a natural sequence or are encoded by substantially a same nucleotide sequence and capable of having a natural sequence. In the context of the present application, the functional active fragment of any given sequence refers to a sequence in which a specific sequence of residues (regardless of amino acid or nucleotide residues) has been modified to retain at least one endogenous function of the polypeptide or polynucleotide. A sequence encoding a functional active fragment can be obtained by adding, deleting, substituting, modifying, replacing, and/or mutating at least one amino acid residue and/or nucleotide residue present in a naturally occurring protein and/or polynucleotide, as long as the original functional activity is maintained.

In the present application, the term "derivative" usually refers to any substitution, mutation, modification, replacement, deletion, and/or addition of one (or more) amino acid residues of a self/pair sequence in the polypeptide or polynucleotide of the present application, as long as the resulting polypeptide or polynucleotide substantially retains at least one of its endogenous functions.

In the present application, the term "analog" usually refers to any analog of a polypeptide or polynucleotide, namely, a chemical compound having at least one endogenous function of the polypeptide or polynucleotide simulated by the analog.

Usually, amino acid substitution, such as substitution by at least one (such as 1, 2, 3, 4, 5, 6, 7, 8, 9, 10, 20, or more) amino acid, may be performed, as long as the modified sequence maintains the desired activity or ability. The amino acid substitution may include use of non-natural analogs.

In the present application, the term "homolog" usually refers to an amino acid sequence or nucleotide sequence that has some homology with a naturally occurring sequence. The term "homology" may be equivalent to the "identity" of the sequence. Homologous sequences may include amino acid sequences that may be at least 80%, 85%, 90%, 99.1%, 99.2%, 99.3%, 99.4%, 99.5%, 99.6%, 99.7%, 99.8%, or 99.9% identical to a subject sequence. Generally, the homolog includes same active sites as the subject amino acid sequence and the like. The homology may be considered based on similarity (namely, amino acid residues having similar chemical properties/functions), or expressed in terms of sequence identity. In the present application, any sequence having percentage identity in the SEQ ID NO of an amino acid or nucleotide sequence mentioned refers to a sequence having the percentage identity throughout the entire length of the SEQ ID NO. In order to determine sequence identity, sequences may be compared in various ways known to those skilled in the art, such as using BLAST, BLAST-2, ALIGN, NEEDLE, or Megalign (DNASTAR) software. Those skilled in the art can determine appropriate parameters for comparison, including any algorithm required to implement optimal comparison in the compared full-length sequence.

The protein or polypeptide used in the present application may also have deletion, insertion, or substitution of amino acid residues, which produce silent changes and result in functionally equivalent proteins. Intentional amino acid substitutions may be made according to similarities in polarity, charge, solubility, hydrophobicity, hydrophilicity, and/or amphoteric properties of residues, as long as endogenous functions are retained. For example, negatively charged amino acids include aspartic acid and glutamic acid; positively charged amino acids include lysine and arginine; and amino acids having similar hydrophilicity values without electropolar head groups include asparagine, glutamine, serine, threonine, and tyrosine.

In the present application, the term "immunoconjugate" usually refers to a conjugate formed by conjugation of other therapeutic agents with the isolated antigen binding protein (such as covalent linking by connecting molecules), where the conjugate can deliver the other therapeutic agents to a target cell through specific binding of the isolated antigen binding protein to an antigen on the target cell. Moreover, the antigen may be secreted by the target cell and located in a gap outside the target cell.

In the present application, the term "subject" usually refers to human or non-human animals, including but not limited to cats, dogs, horses, pigs, cows, sheep, rabbits, mice, rats, or monkeys.

In the present application, the term "nucleic acid molecule" usually refers to any length of isolated form of nucleotide, deoxyribonucleotide, or ribonucleotide isolated from the natural environment or artificially synthesized or analogs thereof.

In the present application, the term "vector" usually refers to a nucleic acid molecule that can transfer another nucleic acid connected thereto. The vector may transfer inserted nucleic acid molecules to cells and/or between cells. The vector may include vectors mainly used for inserting DNA or RNA into cells, vectors mainly used for replicating DNA or RNA, and vectors mainly used for expressing the transcription and/or translation of DNA or RNA. The vector may be a polynucleotide that can be transcribed and translated into a polypeptide when introduced into suitable cells. Usually, by culturing the suitable cells including the vector, the vector can produce a desired expression product. In the present application, the vector may include lentivirus vectors.

In the present application, the term "cell" usually refers to an individual cell, cell line, or cell culture that can or has included a plasmid or vector including the nucleic acid molecule described in the present application, or that can express the polypeptide or antigen binding protein described in the present application. The cell may include offspring of a single cell. Due to natural, accidental or intentional mutations, offspring cells may not necessarily be identical in morphology or genome to original parent cells, but can express the polypeptide or antigen binding protein described in the present application. The cells may be obtained by in vitro transfection of cells using the vector described in the present application. The cells may be prokaryotic cells (such as Escherichia coli) or eukaryotic cells (such as yeast cells, including COS cells, Chinese hamster ovary (CHO) cells, HeLa cells, HEK293 cells, COS-1 cells, NS0 cells, or myeloma cells). In some embodiments, the cells may be immune cells. For example, the immune cells may be selected from the following groups: T cells, B cells, natural killer cells (NK cells), macrophages, NKT cells, monocytes, dendritic cells, granulocytes, lymphocytes, white blood cells, and/or peripheral blood mononuclear cells.

In the present application, the term "treatment" usually refers to: (i) preventing patients who may be susceptible to diseases, illness, and/or conditions but have not yet been diagnosed out from developing the diseases, illness, and/or conditions; (ii) suppressing the diseases, illness, or conditions, namely, suppressing the development thereof; and (iii) alleviating the diseases, illness, or conditions, namely, regressing the diseases, illness, and/or conditions and/or symptoms associated with the diseases, illness, and/or conditions.

In the present application, the terms "polypeptide", "peptide", and "protein" are used interchangeably and usually refer to a polymer of amino acids of any length. The polymer may be linear or branched, include modified amino acids, and be interrupted by non amino acids. These terms further cover amino acid polymers that have been modified. These modifications may include: disulfide bond formation, glycosylation, lipoylation, acetylation, phosphorylation, or any other manipulation (such as binding to labeled components). The term "amino acid" includes natural and/or unnatural or synthetic amino acids, including glycine and D and L optical isomers, as well as amino acid analogs and peptide analogs.

In the present application, the terms "polynucleotide", "nucleotide", "nucleotide sequence", "nucleic acid", and "oligonucleotide" are used interchangeably and usually refer to a polymerization form of nucleotides of any length, such as deoxyribonucleotide or ribonucleotide or analogs thereof. The polynucleotide may have any three-dimensional structure and perform any known or unknown function. The following are non-limiting examples of the polynucleotide: coding or non-coding regions of genes or gene fragments, multiple loci (one locus) defined by linkage analysis, exons, introns, messenger RNA (mRNA), transfer RNA, ribosomal RNA, short interfering RNA (siRNA), short hairpin RNA (shRNA), micro-RNA (miRNA), ribozymes, cDNA, recombinant polynucleotides, branched polynucleotides, plasmids, vectors any sequence of isolated DNA, any sequence of isolated RNA, nucleic acid probes, and primers. The polynucleotide may include one or more modified nucleotides, such as methylated nucleotides and nucleotide analogs. If present, the nucleotide structure may be modified before or after polymer assembly. The nucleotide sequence may be interrupted by non-nucleotide components. The polynucleotide may be further modified after polymerization, such as by conjugating with labeled components.

In the present application, the term "*K_{D}*" (similarly, "*K_{D}*" or "*K_{D}*") usually refers to an "affinity constant" or "equilibrium dissociation constant", and refers to a value obtained at equilibrium in titrimetry or by dividing a dissociation rate constant (kd) by a binding rate constant (ka). The binding rate constant (ka), the dissociation rate constant (kd), and the equilibrium dissociation constant (*K_{D}*) represent the binding affinity of a binding protein (such as the isolated antigen binding protein described in the present application) to an antigen (such as MASP-2 protein). Methods for determining the binding and dissociation rate constants are well-known in the art. Fluorescence-based technologies provide high sensitivity and an ability to check samples at equilibrium in a physiological buffer. For example, the *K_{D}* value can be measured by Biocore (biomolecular interaction analysis) (such as instruments obtained from BIAcore InternationalAB, aGEHealthcare company, Uppsala, Sweden), or other experimental pathways and instruments such as Octet detection. In addition, the *K_{D}* value may also be measured by KinExA (KineticExclusionAssay) obtained from SapidyneInstruments (Boise, Idaho), or a surface plasmon resonator (SPR). For example, the *K_{D}* value may also be measured by an amine coupling kit.

In the present application, the term "and/or" should be understood as any one or two of options.

In the present application, the term "include" usually refers to include clearly specified features, but does not exclude other elements. In some cases, "include" further covers situations where only specified components are included. For example, "include" also represents the meaning of "composed of...".

In the present application, the term "about" usually refers to changes within a range of 0.5% to 10% above or below a specified value, such as changes within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

In the present application, the term "include" usually refers to the meaning of inclusion, summation, containing, or coverage. In some cases, "include" also represents the meaning of "is" or "composed of".

### DETAILED DESCRIPTION OF THE INVENTION

### Isolated antigen binding protein of the present application

In one aspect, the present application provides an isolated antigen binding protein, which can specifically bind to a human MASP-2 protein at a *K_{D}* value of about 2E-09M or less (for example, the *K_{D}* value is not more than about 2E-09M, not more than about 1.5E-09M, not more than about 1E-09M, not more than about 9E-10M, not more than about 8E-10M, not more than about 7E-10M, not more than about 6E-10M, not more than about 5E-10M, not more than about 2E-10M, not more than about 1E-10M, not more than about 5E-11M, not more than about 1E-11M, or not more than 5E-12M or less) in Octet detection.

In one aspect, the present application provides an isolated antigen binding protein, which can specifically bind to a cynomolgus monkey MASP-2 protein at a *K_{D}* value of about 2E-09M or less (for example, the *K_{D}* value is not more than about 2E-09M, not more than about 1.5E-09M, not more than about 1E-09M, not more than about 9E-10M, not more than about 8E-10M, not more than about 7E-10M, not more than about 6E-10M, not more than about 5E-10M, not more than about 2E-10M, not more than about 1E-10M, not more than about 5E-11M, not more than about 1E-11M, or not more than 5E-12M or less) in Octet detection.

In one aspect, the present application provides an isolated antigen binding protein, which may include at least one CDR in a heavy chain variable region VH of an antibody, where the VH may include an amino acid sequence shown in SEQ ID NO: 67 or SEQ ID NO: 77.

For example, the VH may include an amino acid sequence shown in any of SEQ ID NO: 13, 26, 39, and 52. In the present application, the HCDR of the isolated antigen binding protein may be divided in any form. As long as the VH is the same as the amino acid sequence shown in any of SEQ ID NO: 13, 26, 39, and 52, the HCDR obtained in any form can fall within the protection scope of the present application.

The CDR of the antibody, also known as a complementary determining region, is a portion of the variable region. Amino acid residues in the region can be in contact with antigens or antigenic epitopes. The CDR of the antibody can be determined by various coding systems, such as CCG, Kabat, Chothia, IMGT, AbM, and comprehensive consideration of Kabat/Chothia. These coding systems are known in the art and can be found in, for example, http://www.bioinf.org.uk/abs/index.html#kabatnum. Those skilled in the art can determine a CDR region by using different coding systems according to the sequence and structure of an antibody. By using different coding systems, the CDR region may be different. In the present application, the CDR covers CDR sequences obtained by any CDR division or variants thereof, where the variants include one or more amino acids substituted, deleted, and/or added in an amino acid sequence of the CDR, for example, 1-30, 1-20, or 1-10, or 1, 2, 3, 4, 5, 6, 7, 8, or 9 amino acids substituted, deleted, and/or inserted. The CDR also covers homologs, which may be amino acid sequences having at least about 85% (for example, at least about 85%, about 90%, about 91%, about 92%, about 93%, about 94%, about 95%, about 96%, about 97%, about 98%, about 99% or more) of sequence homology with the amino acid sequences of the CDR. In some embodiments, the isolated antigen binding protein described in the present application is defined by a Chothia coding system.

In the present application, the antigen binding protein may comprisea heavy chain variable region VH, and the VH may compriseat least one, two, or three of HCDR1, HCDR2, and HCDR3.

In the present application, the HCDR3 of the antigen binding protein may include an amino acid sequence shown in SEQ ID NO: 19. For example, the HCDR3 sequence of the antigen binding protein may be defined according to the Chothia coding system.

In the present application, the HCDR2 of the antigen binding protein may include an amino acid sequence shown in SEQ ID NO: 17. For example, the HCDR2 sequence of the antigen binding protein may be defined according to the Chothia coding system.

In the present application, the HCDR1 of the antigen binding protein may include an amino acid sequence shown in SEQ ID NO: 15. For example, the HCDR1 sequence of the antigen binding protein may be defined according to the Chothia coding system.

For example, the HCDR1 of the antigen binding protein may comprise an amino acid sequence shown in SEQ ID NO: 15; the HCDR2 may include an amino acid sequence shown in SEQ ID NO: 17; and the HCDR3 may include an amino acid sequence shown in SEQ ID NO: 19. For example, the antigen binding protein may include an antibody 50A6, JYB1931A63, or an antigen binding fragment having the same HCDR3 (for example, having the same HCDR1-3).

For example, the VH of the antigen binding protein may include framework regions H-FR1, H-FR2, H-FR3, and H-FR4.

In the present application, the H-FR1 of the antigen binding protein may comprise an amino acid sequence shown in SEQ ID NO: 62. For example, compared to the sequence shown in SEQ ID NO: 62, the H-FR1 of the antigen binding protein has amino acid substituents (such as conserved amino acid substituents) at one or more amino acids selected from the following group: X₁₆ and X₁₉.

EVQLVESGGGLVQPG X₁₆SL X₁₉LSCAAS (SEQ ID NO: 62), where X₁₆ may be G or R, and X₁₉ may be R or S.

In the present application, the H-FR1 of the antigen binding protein may comprise an amino acid sequence shown in any of SEQ ID NO: 14 and SEQ ID NO: 27.

In the present application, the H-FR2 of the antigen binding protein may comprise an amino acid sequence shown in SEQ ID NO: 63. For example, compared to the sequence shown in SEQ ID NO: 63, the H-FR2 of the antigen binding protein has amino acid substituents (such as conserved amino acid substituents) at one or more amino acids selected from the following group: X₈ and X₁₀.

NMAWVRQ X₈P X₁₀KGLEWVATI (SEQ ID NO: 63), where X₈ may be A or T, and X₁₀ may be G or K.

In the present application, the H-FR2 of the antigen binding protein may comprise an amino acid sequence shown in any of SEQ ID NO: 16 and SEQ ID NO: 28.

In the present application, the H-FR3 of the antigen binding protein may comprise an amino acid sequence shown in SEQ ID NO: 64. For example, compared to the sequence shown in SEQ ID NO: 64, the H-FR3 of the antigen binding protein has amino acid substituents (such as conserved amino acid substituents) at one or more amino acids selected from the following group: X₁₆, X₂₀, X₂₁, X₂₇, X₃₁, and X₃₆.

TYYRDSVKGRFTISR X₁₆NAK X₂₀ X₂₁LYLQM X₂₇SLR X₃₁EDTA X₃₆YYCST (SEQ ID NO: 64), where X₁₆ may be D or E, X₂₀ may be N or S, X₂₁ may be S or T, X₂₇ may be D or N, X₃₁ may be A or S, and X₃₆ may be T or V.

In the present application, the H-FR3 of the antigen binding protein may comprise an amino acid sequence shown in any of SEQ ID NO: 18 and SEQ ID NO: 29.

In the present application, the H-FR4 of the antigen binding protein may comprise an amino acid sequence shown in SEQ ID NO: 65. For example, compared to the sequence shown in SEQ ID NO: 65, the H-FR4 of the antigen binding protein has amino acid substituents (such as conserved amino acid substituents) at one or more amino acids selected from the following group: X₅ and X₆.

WGQG X₅ X₆VTVSS (SEQ ID NO: 65), where X₅ may be T or V, and X₆ may be L or M.

In the present application, the H-FR4 of the antigen binding protein may comprise an amino acid sequence shown in any of SEQ ID NO: 20 and SEQ ID NO: 30.

In the present application, the H-FR1 of the antigen binding protein may comprise an amino acid sequence shown in SEQ ID NO: 62; the H-FR2 may comprise an amino acid sequence shown in SEQ ID NO: 63; the H-FR3 may comprise an amino acid sequence shown in SEQ ID NO: 64; and the H-FR4 may comprise an amino acid sequence shown in SEQ ID NO: 65.

In the present application, the H-FR1 of the antigen binding protein may comprise an amino acid sequence shown in any of SEQ ID NO: 14 and SEQ ID NO: 27; the H-FR2 may comprise an amino acid sequence shown in any of SEQ ID NO: 16 and SEQ ID NO: 28; the H-FR3 may comprise an amino acid sequence shown in any of SEQ ID NO: 18 and SEQ ID NO: 29; and the H-FR4 may comprise an amino acid sequence shown in any of SEQ ID NO: 20 and SEQ ID NO: 30.

In the present application, the H-FR1 of the antigen binding protein may comprise an amino acid sequence shown in SEQ ID NO: 14; the H-FR2 may comprise an amino acid sequence shown in SEQ ID NO: 16; the H-FR3 may comprise an amino acid sequence shown in SEQ ID NO: 18; and the H-FR4 may comprise an amino acid sequence shown in SEQ ID NO: 20. For example, the antigen binding protein may comprise an antibody 50A6 or an antigen binding fragment having the same H-FR1-4.

In the present application, the H-FR1 of the antigen binding protein may comprise an amino acid sequence shown in SEQ ID NO: 27; the H-FR2 may comprise an amino acid sequence shown in SEQ ID NO: 28; the H-FR3 may comprise an amino acid sequence shown in SEQ ID NO: 29; and the H-FR4 may comprise an amino acid sequence shown in SEQ ID NO: 30. For example, the antigen binding protein may comprise an antibody JYB1931A63 or an antigen binding fragment having the same H-FR1-4.

In the present application, the antigen binding protein may comprise a heavy chain variable region, and the heavy chain variable region may comprise an amino acid sequence shown in SEQ ID NO: 67. For example, the antigen binding protein comprises a VH, and compared to the sequence shown in SEQ ID NO: 67, the VH has amino acid substituents (such as conserved amino acid substituents) at one or more amino acids selected from the following group: X₁₆, X₁₉, X₄₀, X₄₂, X₇₃, X₇₇, X₇₈, X₈₄, X₈₈, X₉₃, X₁₁₉, and X₁₂₀.

EVQLVESGGGLVQPGX₁₆SLX₁₉LSCAASGFTFNDYNMAWVRQX₄₀PX₄₂KGLE WVATILFDGSRTYYRDSVKGRFTISRX₇₃NAKX₇₇X₇₈LYLQMX₈₄SLRX₈₈EDTAX₉₃YY CSTESPYYSEGYYQGYFDYWGQG X₁₁₉ X₁₂₀VTVSS (SEQ ID NO: 67), where X₁₆ may be G or R, X₁₉ may be R or S, X₄₀ may be A or T, X₄₂ may be G or K, X₇₃ may be D or E, X₇₇ may be N or S, X₇₈ may be S or T, X₈₄ may be D or N, X₈₈ may be A or S, X₉₃ may be T or V, X₁₁₉ may be T or V, and X₁₂₀ may be L or M.

In the present application, the heavy chain variable region of the antigen binding protein may comprise an amino acid sequence shown in any of SEQ ID NO: 13 and SEQ ID NO: 26.

In the present application, the antigen binding protein may comprise a heavy chain constant region, and the heavy chain constant region may comprise a constant region derived from IgG or a constant region derived from IgY.

For example, the heavy chain constant region of the antigen binding protein may comprise an amino acid sequence shown in SEQ ID NO: 56.

In the present application, the antigen binding protein may comprise at least one CDR in a light chain variable region VL of the antibody, and the VL may comprise an amino acid sequence shown in SEQ ID NO: 66 or SEQ ID NO: 76.

For example, the VL may comprise an amino acid sequence shown in either SEQ ID NO: 66 or SEQ ID NO: 76. In the present application, the LCDR of the isolated antigen binding protein may be divided in any form. As long as the VL is the same as the amino acid sequence shown in either SEQ ID NO: 66 or SEQ ID NO: 76, the LCDR divided in any form can fall within the protection scope of the present application.

In the present application, the antigen binding protein may comprise a light chain variable region VL, and the VL may comprise at least one, at least two, or at least three of LCDR1, LCDR2, and LCDR3.

In the present application, the LCDR3 of the antigen binding protein may comprise an amino acid sequence shown in SEQ ID NO: 11. For example, the LCDR3 of the antigen binding protein may be defined according to a Chothia numbering system.

In the present application, the LCDR2 of the antigen binding protein may comprise an amino acid sequence shown in SEQ ID NO: 9. For example, the LCDR2 of the antigen binding protein may be defined according to a Chothia numbering system.

In the present application, the LCDR1 of the antigen binding protein may comprise an amino acid sequence shown in SEQ ID NO: 7. For example, the LCDR1 of the antigen binding protein may be defined according to the Chothia numbering system.

For example, the LCDR1 of the antigen binding protein described in the present application may comprise an amino acid sequence shown in SEQ ID NO: 7; the LCDR2 may comprise an amino acid sequence shown in SEQ ID NO: 9; and the LCDR3 may comprise an amino acid sequence shown in SEQ ID NO: 11. For example, the antigen binding protein may comprise an antibody 50A6, JYB1931A63, or an antigen binding fragment having the same LCDR3 (for example, having the same LCDR1-3).

For example, the VL of the antigen binding protein may comprise framework regions L-FR1, L-FR2, L-FR3, and L-FR4.

In the present application, the L-FR1 of the antigen binding protein may comprise an amino acid sequence shown in SEQ ID NO: 58. For example, compared to the sequence shown in SEQ ID NO: 58, the L-FR1 of the antigen binding protein has amino acid substituents (such as conserved amino acid substituents) at one or more amino acids selected from the following group: X₁, X₁₃, X₁₄, X₁₈, X₁₉, and X₂₀.

X₁IVLTQSPATLSX₁₃X₁₄PGE X₁₈X₁₉X₂₀LSC (SEQ ID NO: 58), where X₁ may be E or N, X₁₃ may be L or V, X₁₄ may be S or T, X₁₈ may be R or S, X₁₉ may be A or V, and X₂₀ may be S or T.

In the present application, the L-FR1 of the antigen binding protein may comprise an amino acid sequence shown in any of SEQ ID NO: 6 and SEQ ID NO: 22.

In the present application, the L-FR2 of the antigen binding protein may comprise an amino acid sequence shown in SEQ ID NO: 59. For example, compared to the sequence shown in SEQ ID NO: 59, the L-FR2 of the antigen binding protein has amino acid substituents (such as conserved amino acid substituents) at one or more amino acids selected from the following group: X₅, X₆, X₇, X₈, and X₉.

WYQQ X₅X₆X₇X₈X₉PRLLIK (SEQ ID NO: 59), where X₅ may be K or R, X₆ may be P or S, X₇ may be G or N, X₈ may be E or Q, and X₉ may be A or S.

In the present application, the L-FR2 of the antigen binding protein may comprise an amino acid sequence shown in any of SEQ ID NO: 8 and SEQ ID NO: 23.

In the present application, the L-FR3 of the antigen binding protein may comprise an amino acid sequence shown in SEQ ID NO: 60. For example, compared to the sequence shown in SEQ ID NO: 60, the L-FR3 of the antigen binding protein has amino acid substituents (such as conserved amino acid substituents) at one or more amino acids selected from the following group: X₄, X₁₈, X₂₀, X₂₁, X₂₂, X₂₄, X₂₈, and X₂₉.

GIPX₄RFSGSGSGTDFTLX₁₈I X₂₀X₂₁X₂₂EX₂₄EDFX₂₈X₂₉YYC (SEQ ID NO: 60), where X₄ may be A or S, X₁₈ may be S or T, X₂₀ may be N or S, X₂₁ may be R or S, X₂₂ may be L or V, X₂₄ may be P or S, X₂₈ may be A or S, and X₂₉ may be I or V.

In the present application, the L-FR3 of the antigen binding protein may comprise an amino acid sequence shown in any of SEQ ID NO: 10 and SEQ ID NO: 24.

In the present application, the L-FR4 of the antigen binding protein may comprise an amino acid sequence shown in SEQ ID NO: 61. For example, compared to the sequence shown in SEQ ID NO: 61, the L-FR4 of the antigen binding protein has amino acid substituents (such as conserved amino acid substituents) at one or more amino acids selected from the following group: X₃, X₉, and X₁₀.

FGX₃GTKLE X₉ X₁₀ (SEQ ID NO: 61), where X₃ may be A or Q, X₉ may be I or L, and X₁₀ may be K or R.

In the present application, the L-FR4 of the antigen binding protein may comprise an amino acid sequence shown in any of SEQ ID NO: 12 and SEQ ID NO: 25.

In the present application, the L-FR1 of the antigen binding protein may comprise an amino acid sequence shown in SEQ ID NO: 58; the L-FR2 may comprise an amino acid sequence shown in SEQ ID NO: 59; the L-FR3 may comprise an amino acid sequence shown in SEQ ID NO: 60; and the L-FR4 may comprise an amino acid sequence shown in SEQ ID NO: 61.

In the present application, the L-FR1 of the antigen binding protein may comprise an amino acid sequence shown in any of SEQ ID NO: 6 and SEQ ID NO: 22; the L-FR2 may comprise an amino acid sequence shown in any of SEQ ID NO: 8 and SEQ ID NO: 23; the L-FR3 may comprise an amino acid sequence shown in any of SEQ ID NO: 10 and SEQ ID NO: 24; and the L-FR4 may comprise an amino acid sequence shown in any of SEQ ID NO: 12 and SEQ ID NO: 25.

In the present application, the L-FR1 of the antigen binding protein may comprise an amino acid sequence shown in SEQ ID NO: 6; the L-FR2 may comprise an amino acid sequence shown in SEQ ID NO: 8; the L-FR3 may comprise an amino acid sequence shown in SEQ ID NO: 10; and the L-FR4 may comprise an amino acid sequence shown in SEQ ID NO: 12. For example, the antigen binding protein may comprise an antibody 50A6 or an antibody having the same L-FR1-4.

In the present application, the L-FR1 of the antigen binding protein may comprise an amino acid sequence shown in SEQ ID NO: 22; the L-FR2 may comprise an amino acid sequence shown in SEQ ID NO: 23; the L-FR3 may comprise an amino acid sequence shown in SEQ ID NO: 24; and the L-FR4 may comprise an amino acid sequence shown in SEQ ID NO: 25. For example, the antigen binding protein may comprise an antibody JYB1931A63 or an antibody having the same H-FR1-4.

In the present application, the antigen binding protein may comprise a light chain variable region VL, and the VL may comprise an amino acid sequence shown in SEQ ID NO: 66. For example, compared to the sequence shown in SEQ ID NO: 66, the VL of the antigen binding protein has amino acid substituents (such as conserved amino acid substituents) at one or more amino acids selected from the following group: X₁, X₁₃, X₁₄, X₁₈, X₁₉, X₂₀, X₃₉, X₄₀, X₄₁, X₄₂, X₄₃, X₆₀, X₇₄, X₇₆, X₇₇, X₇₈, X₈₀, X₈₄, X₈₅, X₁₀₀, X₁₀₆, and X₁₀₇. X₁IVLTQSPATLSX₁₃X₁₄PGEX₁₈X₁₉X₂₀LSCRASQSVSTSIHWYQQX₃₉X₄₀X₄₁X₄₂X ₄₃PRLLIKYASRSISGIPX₆₀RFSGSGSGTDFTLX₇₄IX₇₆X₇₇X₇₈EX₈₀EDFX₈₄X₈₅YYCQQS YSSLYTFGX₁₀₀GTKLEX₁₀₆ X₁₀₇ (SEQ ID NO: 66), where X₁ may be E or N, X₁₃ may be L or V, X₁₄ may be S or T, X₁₈ may be R or S, X₁₉ may be A or V, X₂₀ may be S or T, X₃₉ may be K or R, X₄₀ may be P or S, X₄₁ may be G or N, X₄₂ may be E or Q, X₄₃ may be A or S, X₆₀ may be A or S, X₇₄ may be S or T, X₇₆ may be N or S, X₇₇ may be R or S, X₇₈ may be L or V, X₈₀ may be P or S, X₈₄ may be A or S, X₈₅ may be I or V, X₁₀₀ may be A or Q, X₁₀₆ may be I or L, and X₁₀₇ may be K or R.

In the present application, the light chain variable region of the antigen binding protein may comprise an amino acid sequence shown in any of SEQ ID NO: 5 and SEQ ID NO: 21.

In the present application, the antigen binding protein may comprise a light chain constant region, and the light chain constant region may comprise a constant region derived from Igκ or a constant region derived from Igλ.

For example, the light chain constant region may comprise a constant region derived from Igκ.

For example, the light chain constant region of the antigen binding protein comprises an amino acid sequence shown in SEQ ID NO: 57.

In the present application, the antigen binding protein may comprise HCDR1-3 and LCDR1-3. For example, the HCDR1 of the antigen binding protein may comprise an amino acid sequence shown in SEQ ID NO: 15; the HCDR2 of the antigen binding protein may comprise an amino acid sequence shown in SEQ ID NO: 17; the HCDR3 of the antigen binding protein may comprise an amino acid sequence shown in SEQ ID NO: 19; the LCDR1 of the antigen binding protein may comprise an amino acid sequence shown in SEQ ID NO: 7; the LCDR2 of the antigen binding protein may comprise an amino acid sequence shown in SEQ ID NO: 9; and the LCDR3 of the antigen binding protein may comprise an amino acid sequence shown in SEQ ID NO: 11. For example, the antigen binding protein may comprise an antibody 50A6, JYB1931A63, or an antigen binding fragment having the same HCDR3 (for example, having the same HCDR1-3) and LCDR3 (for example, having the same LCDR1-3).

In the present application, the antigen binding protein may comprise a heavy chain variable region and a light chain variable region. The heavy chain variable region of the antigen binding protein may comprise HCDR1-3 and H-FR1-4. The light chain variable region of the antigen binding protein may comprise LCDR1-3 and L-FR1-4. For example, the HCDR1 may comprise an amino acid sequence shown in SEQ ID NO: 15; the HCDR2 may comprise an amino acid sequence shown in SEQ ID NO: 17; the HCDR3 may comprise an amino acid sequence shown in SEQ ID NO: 19; the LCDR1 may comprise an amino acid sequence shown in SEQ ID NO: 7; the LCDR2 may comprise an amino acid sequence shown in SEQ ID NO: 9; and the LCDR3 may comprise an amino acid sequence shown in SEQ ID NO: 11. For example, the H-FR1 may comprise an amino acid sequence shown in SEQ ID NO: 14; the H-FR2 may comprise an amino acid sequence shown in SEQ ID NO: 16; the H-FR3 may comprise an amino acid sequence shown in SEQ ID NO: 18; the H-FR4 may comprise an amino acid sequence shown in SEQ ID NO: 20; the L-FR1 may comprise an amino acid sequence shown in SEQ ID NO: 6; the L-FR2 may comprise an amino acid sequence shown in SEQ ID NO: 8; the L-FR3 may comprise an amino acid sequence shown in SEQ ID NO: 10; and the L-FR4 may comprise an amino acid sequence shown in SEQ ID NO: 12. For example, the heavy chain variable region of the antigen binding protein may comprise an amino acid sequence shown in SEQ ID NO: 13. For example, the antigen binding protein may comprise an antibody 50A6 or an antigen binding protein having the same heavy chain variable region. For example, the light chain variable region of the antigen binding protein may comprise an amino acid sequence shown in SEQ ID NO: 5. For example, the antigen binding protein may comprise an antibody 50A6 or an antigen binding protein having the same light chain variable region.

In the present application, the antigen binding protein may comprise a heavy chain variable region and a light chain variable region, and the heavy chain variable region may comprise HCDR1-3 and H-FR1-4. The light chain variable region may comprise LCDR1-3 and L-FR1-4. For example, the HCDR1 may comprise an amino acid sequence shown in SEQ ID NO: 15; the HCDR2 may comprise an amino acid sequence shown in SEQ ID NO: 17; the HCDR3 may comprise an amino acid sequence shown in SEQ ID NO: 19; the LCDR1 may comprise an amino acid sequence shown in SEQ ID NO: 7; the LCDR2 may comprise an amino acid sequence shown in SEQ ID NO: 9; and the LCDR3 may comprise an amino acid sequence shown in SEQ ID NO: 11. For example, the H-FR1 may comprise an amino acid sequence shown in SEQ ID NO: 27; the H-FR2 may comprise an amino acid sequence shown in SEQ ID NO: 28; the H-FR3 may comprise an amino acid sequence shown in SEQ ID NO: 29; the H-FR4 may comprise an amino acid sequence shown in SEQ ID NO: 30; the L-FR1 may comprise an amino acid sequence shown in SEQ ID NO: 22; the L-FR2 may comprise an amino acid sequence shown in SEQ ID NO: 23; the L-FR3 may comprise an amino acid sequence shown in SEQ ID NO: 24; and the L-FR4 may comprise an amino acid sequence shown in SEQ ID NO: 25. For example, the heavy chain variable region of the antigen binding protein may comprise an amino acid sequence shown in SEQ ID NO: 26. For example, the antigen binding protein may comprise an antibody JYB1931A63 or an antigen binding protein having the same heavy chain variable region. For example, the light chain variable region of the antigen binding protein may comprise an amino acid sequence shown in SEQ ID NO: 21. For example, the antigen binding protein may comprise an antibody JYB1931A63 or an antigen binding protein having the same light chain variable region.

In the present application, the antigen binding protein may comprise a heavy chain variable region VH, and the VH may comprise at least one, two, or three of HCDR1, HCDR2, and HCDR3.

In the present application, the HCDR3 of the antigen binding protein may comprise an amino acid sequence shown in SEQ ID NO: 45. For example, the HCDR3 sequence of the antigen binding protein may be defined according to the Chothia coding system.

In the present application, the HCDR2 of the antigen binding protein may comprise an amino acid sequence shown in SEQ ID NO: 43. For example, the HCDR2 sequence of the antigen binding protein may be defined according to the Chothia coding system.

In the present application, the HCDR1 of the antigen binding protein may comprise an amino acid sequence shown in SEQ ID NO: 41. For example, the HCDR1 sequence of the antigen binding protein may be defined according to the Chothia coding system.

For example, the HCDR1 of the antigen binding protein may comprise an amino acid sequence shown in SEQ ID NO: 41; the HCDR2 may comprise an amino acid sequence shown in SEQ ID NO: 43; and the HCDR3 may comprise an amino acid sequence shown in SEQ ID NO: 45. For example, the antigen binding protein may comprise an antibody 47A1, JYB1931A13, or an antigen binding fragment having the same HCDR3 (for example, having the same HCDR1-3).

For example, the VH of the antigen binding protein may comprise framework regions H-FR1, H-FR2, H-FR3, and H-FR4.

In the present application, the H-FR1 of the antigen binding protein may comprise an amino acid sequence shown in SEQ ID NO: 72. For example, compared to the sequence shown in SEQ ID NO: 72, the H-FR1 of the antigen binding protein has amino acid substituents (such as conserved amino acid substituents) at one or more amino acids selected from the following group: X1, X₁₇, and X₂₅.

X₁VQLQESGPGLVKPSQX₁₇LSLTCTVX₂₅ (SEQ ID NO: 72), where X₁ may be D or Q, X₁₇ may be S or T, and X₂₅ may be S or T.

In the present application, the H-FR1 of the antigen binding protein may comprise an amino acid sequence shown in any of SEQ ID NO: 40 and SEQ ID NO: 53.

In the present application, the H-FR2 of the antigen binding protein may comprise an amino acid sequence shown in SEQ ID NO: 73. For example, compared to the sequence shown in SEQ ID NO: 73, the H-FR2 of the antigen binding protein has amino acid substituents (such as conserved amino acid substituents) at one or more amino acids selected from the following group: X₈, X₁₁, X₁₂, and X₁₆.

AWNWIRQ X₈PGX₁₁X₁₂LEWX₁₆GYI (SEQ ID NO: 73), where X₈ may be F or P, X₁₁ may be K or N, X₁₂ may be G or K, and X₁₆ may be I or M.

In the present application, the H-FR2 of the antigen binding protein may comprise an amino acid sequence shown in any of SEQ ID NO: 42 and SEQ ID NO: 54.

In the present application, the H-FR3 of the antigen binding protein may comprise an amino acid sequence shown in SEQ ID NO: 74. For example, compared to the sequence shown in SEQ ID NO: 74, the H-FR3 of the antigen binding protein has amino acid substituents (such as conserved amino acid substituents) at one or more amino acids selected from the following group: X₁₁, X₁₂, X₁₄, X₁₆, X₁₉, X₂₃, X₂₅, X₂₇, X₃₁, X₃₂, X₃₆, and X₃₈.

TSYNPSLKSR X₁₁ X₁₂I X₁₄RX₁₆TSX₁₉NQF X₂₃LX₂₅LX₂₇SVTX₃₁X₃₂DTAX₃₆YX₃₈CAR (SEQ ID NO: 74),where X₁₁ may be I or V, X₁₂ may be S or T, X₁₄ may be S or T, X₁₆ may be D or N, X₁₉ may be K or T, X₂₃ may be F or S, X₂₅ may be K or Q, X₂₇ may be N or S, X₃₁ may be A or T, X₃₂ may be A or E, X₃₆ may be T or V, and X₃₈ may be F or Y.

In the present application, the H-FR3 of the antigen binding protein may comprise an amino acid sequence shown in any of SEQ ID NO: 44 and SEQ ID NO: 55.

In the present application, the H-FR4 of the antigen binding protein may comprise an amino acid sequence shown in SEQ ID NO: 75. For example, compared to the sequence shown in SEQ ID NO: 75, the H-FR4 of the antigen binding protein has amino acid substituents (such as conserved amino acid substituents) at one or more amino acids selected from the following group: X₆ and X₇.

WGQGTX₆X₇TVSS (SEQ ID NO: 75), where X₆ may be L or T, and X₇ may be L or V.

In the present application, the H-FR4 of the antigen binding protein may comprise an amino acid sequence shown in any of SEQ ID NO: 46 and SEQ ID NO: 30.

In the present application, the H-FR1 of the antigen binding protein may comprise an amino acid sequence shown in SEQ ID NO: 72; the H-FR2 may comprise an amino acid sequence shown in SEQ ID NO: 73; the H-FR3 may comprise an amino acid sequence shown in SEQ ID NO: 74; and the H-FR4 may comprise an amino acid sequence shown in SEQ ID NO: 75.

In the present application, the H-FR1 of the antigen binding protein may comprise an amino acid sequence shown in any of SEQ ID NO: 40 and SEQ ID NO: 53; the H-FR2 may comprise an amino acid sequence shown in any of SEQ ID NO: 42 and SEQ ID NO: 54; the H-FR3 may comprise an amino acid sequence shown in any of SEQ ID NO: 44 and SEQ ID NO: 55; and the H-FR4 may comprise an amino acid sequence shown in any of SEQ ID NO: 46 and SEQ ID NO: 30.

In the present application, the H-FR1 of the antigen binding protein may comprise an amino acid sequence shown in SEQ ID NO: 40; the H-FR2 may comprise an amino acid sequence shown in SEQ ID NO: 42; the H-FR3 may comprise an amino acid sequence shown in SEQ ID NO: 44; and the H-FR4 may comprise an amino acid sequence shown in SEQ ID NO: 46. For example, the antigen binding protein may comprise an antibody 47A1 or an antigen binding fragment having the same H-FR1-4.

In the present application, the H-FR1 of the antigen binding protein may comprise an amino acid sequence shown in SEQ ID NO: 53; the H-FR2 may comprise an amino acid sequence shown in SEQ ID NO: 54; the H-FR3 may comprise an amino acid sequence shown in SEQ ID NO: 55; and the H-FR4 may comprise an amino acid sequence shown in SEQ ID NO: 30. For example, the antigen binding protein may comprise an antibody JYB1931A13 or an antigen binding fragment having the same H-FR1-4.

In the present application, the antigen binding protein may comprise a heavy chain variable region, and the heavy chain variable region may comprise an amino acid sequence shown in SEQ ID NO: 77. For example, the antigen binding protein comprises a VH, and compared to the sequence shown in SEQ ID NO: 77, the VH has amino acid substituents (such as conserved amino acid substituents) at one or more amino acids selected from the following group: X₁, X₁₇, X₂₅, X₄₁, X₄₄, X₄₅, X₄₉, X₆₈, X₆₉, X₇₁, X₇₃, X₇₆, X₈₀, X₈₂, X₈₄, X₈₈, X₈₉, X₉₃, X₉₅, X₁₀₉, and X₁₁₀.

X₁VQLQESGPGLVKPSQX₁₇LSLTCTVX₂₅GYSITSDYAWNWIRQX₄₁PG X₄₄X₄₅LEW X₄₉GYISYSGRTSYNPSLKSRX₆₈X₆₉IX₇₁R X₇₃TSX₇₆NQFX₈₀L X₈₂LX₈₄SVTX₈₈X₈₉DTA X₉₃Y X₉₅CARYWGDYWGQGTX₁₀₉X₁₁₀TVSS (SEQ ID NO: 77), where X₁ may be D or Q, X₁₇ may be S or T, X₂₅ may be S or T, X₄₁ may be F or P, X₄₄ may be K or N, X₄₅ may be G or K, X₄₉ may be I or M, X₆₈ may be I or V, X₆₉ may be S or T, X₇₁ may be S or T, X₇₃ may be D or N, X₇₆ may be K or T, X₈₀ may be F or S, X₈₂ may be K or Q, X₈₄ may be N or S, X₈₈ may be A or T, X₈₉ may be A or E, X₉₃ may be T or V, X₉₅ may be F or Y, X₁₀₉ may be L or T, and X₁₁₀ may be L or V.

In the present application, the heavy chain variable region of the antigen binding protein may comprise an amino acid sequence shown in any of SEQ ID NO: 39 and SEQ ID NO: 52.

In the present application, the antigen binding protein may comprise a heavy chain constant region, and the heavy chain constant region may comprise a constant region derived from IgG or a constant region derived from IgY.

For example, the heavy chain constant region of the antigen binding protein may comprise an amino acid sequence shown in SEQ ID NO: 56.

In the present application, the antigen binding protein may comprise a light chain variable region VL, and the VL may comprise at least one, at least two, or at least three of LCDR1, LCDR2, and LCDR3.

In the present application, the LCDR3 of the antigen binding protein may comprise an amino acid sequence shown in SEQ ID NO: 37. For example, the LCDR3 of the antigen binding protein may be defined according to the Chothia numbering system.

In the present application, the LCDR2 of the antigen binding protein may comprise an amino acid sequence shown in SEQ ID NO: 35. For example, the LCDR2 of the antigen binding protein may be defined according to the Chothia numbering system.

In the present application, the LCDR1 of the antigen binding protein may comprise an amino acid sequence shown in SEQ ID NO: 33. For example, the LCDR1 of the antigen binding protein may be defined according to the Chothia numbering system.

For example, the LCDR1 of the antigen binding protein described in the present application may comprise an amino acid sequence shown in SEQ ID NO: 33; the LCDR2 may comprise an amino acid sequence shown in SEQ ID NO: 35; and the LCDR3 may comprise an amino acid sequence shown in SEQ ID NO: 37. For example, the antigen binding protein may comprise an antibody 47A1 or JYB1931A13 or an antigen binding fragment having the same LCDR3 (for example, having the same LCDR1-3).

For example, the VL of the antigen binding protein may comprise framework regions L-FR1, L-FR2, L-FR3, and L-FR4.

In the present application, the L-FR1 of the antigen binding protein may comprise an amino acid sequence shown in SEQ ID NO: 68. For example, compared to the sequence shown in SEQ ID NO: 68, the L-FR1 of the antigen binding protein has amino acid substituents (such as conserved amino acid substituents) at one or more amino acids selected from the following group: X₃, X₈, X₉, X₁₀, X₁₁, X₁₃, X₂₀, and X₂₁.

DIX₃MTQSX₈X₉X₁₀X₁₁S X₁₃SVGDRVX₂₀X₂₁TC (SEQ ID NO: 68), where X₃ may be Q or V, X₈ may be P or Q, X₉ may be K or S, X₁₀ may be F or S, X₁₁ may be L or M, X₁₃ may be A or T, X₂₀ may be S or T, and X₂₁ may be I or V.

In the present application, the L-FR1 of the antigen binding protein may comprise an amino acid sequence shown in any of SEQ ID NO: 32 and SEQ ID NO: 48.

In the present application, the L-FR2 of the antigen binding protein may comprise an amino acid sequence shown in SEQ ID NO: 69. For example, compared to the sequence shown in SEQ ID NO: 69, the L-FR2 of the antigen binding protein has amino acid substituents (such as conserved amino acid substituents) at one or more amino acids selected from the following group: X₈ and X₉.

WFQQKPG X₈ X₉PKPLIY (SEQ ID NO: 69), where X₈ may be K or Q, and X₉ may be A or S.

In the present application, the L-FR2 of the antigen binding protein may comprise an amino acid sequence shown in any of SEQ ID NO: 34 and SEQ ID NO: 49.

In the present application, the L-FR3 of the antigen binding protein may comprise an amino acid sequence shown in SEQ ID NO: 70. For example, compared to the sequence shown in SEQ ID NO: 70, the L-FR3 of the antigen binding protein has amino acid substituents (such as conserved amino acid substituents) at one or more amino acids selected from the following group: X₄, X₇, X₂₁, X₂₂, X₂₄, X₂₇, and X₂₉.

GVP X₄RFX₇GSGSGTDFTLTIS X₂₁X₂₂QX₂₄EDX₂₇A X₂₉YFC (SEQ ID NO: 70), where X₄ may be D or S, X₇ may be S or T, X₂₁ may be N or S, X₂₂ may be L or V, X₂₄ may be P or S, X₂₇ may be F or L, and X₂₉ may be E or T.

In the present application, the L-FR3 of the antigen binding protein may comprise an amino acid sequence shown in any of SEQ ID NO: 36 and SEQ ID NO: 50.

In the present application, the L-FR4 of the antigen binding protein may comprise an amino acid sequence shown in SEQ ID NO: 71. For example, compared to the sequence shown in SEQ ID NO: 71, the L-FR4 of the antigen binding protein has amino acid substituents (such as conserved amino acid substituents) at one or more amino acids selected from the following group: X₃, X₇, X₉, and X₁₀.

FGX₃GTKX₇EX₉X₁₀ (SEQ ID NO: 71), where X₃ may be A or G, X₇ may be L or V, X₉ may be I or L, and X₁₀ may be K or N.

In the present application, the L-FR4 of the antigen binding protein may comprise an amino acid sequence shown in any of SEQ ID NO: 38 and SEQ ID NO: 51.

In the present application, the L-FR1 of the antigen binding protein may comprise an amino acid sequence shown in SEQ ID NO: 68; the L-FR2 may comprise an amino acid sequence shown in SEQ ID NO: 69; the L-FR3 may comprise an amino acid sequence shown in SEQ ID NO: 70; and the L-FR4 may comprise an amino acid sequence shown in SEQ ID NO: 71.

In the present application, the L-FR1 of the antigen binding protein may comprise an amino acid sequence shown in any of SEQ ID NO: 32 and SEQ ID NO: 48; the L-FR2 may comprise an amino acid sequence shown in any of SEQ ID NO: 34 and SEQ ID NO: 49; the L-FR3 may comprise an amino acid sequence shown in any of SEQ ID NO: 36 and SEQ ID NO: 50; and the L-FR4 may comprise an amino acid sequence shown in any of SEQ ID NO: 38 and SEQ ID NO: 51.

In the present application, the L-FR1 of the antigen binding protein may comprise an amino acid sequence shown in SEQ ID NO: 32; the L-FR2 may comprise an amino acid sequence shown in SEQ ID NO: 34; the L-FR3 may comprise an amino acid sequence shown in SEQ ID NO: 36; and the L-FR4 may comprise an amino acid sequence shown in SEQ ID NO: 38. For example, the antigen binding protein may comprise an antibody 47A1 or an antibody having the same L-FR1-4.

In the present application, the L-FR1 of the antigen binding protein may comprise an amino acid sequence shown in SEQ ID NO: 48; the L-FR2 may comprise an amino acid sequence shown in SEQ ID NO: 49; the L-FR3 may comprise an amino acid sequence shown in SEQ ID NO: 50; and the L-FR4 may comprise an amino acid sequence shown in SEQ ID NO: 51. For example, the antigen binding protein may comprise an antibody JYB1931A13 or an antibody having the same H-FR1-4.

In the present application, the antigen binding protein may comprise a light chain variable region VL, and the VL may comprise an amino acid sequence shown in SEQ ID NO: 76. For example, compared to the sequence shown in SEQ ID NO: 76, the VL of the antigen binding protein has amino acid substituents (such as conserved amino acid substituents) at one or more amino acids selected from the following group: X₃, X₈, X₉, X₁₀, X₁₁, X₁₃, X₂₀, X₂₁, X₄₂, X₄₃, X₆₀, X₆₃, X₇₇, X₇₈, X₈₀, X₈₃, X₈₅, X₁₀₀, X₁₀₄, X₁₀₆, and X₁₀₇.

DIX₃MTQSX₈X₉X₁₀X₁₁SX₁₃SVGDRVX₂₀X₂₁TCKASQNVGSNVAWFQQKPGX₄₂X ₄₃PKPLIYSASYRYSGVPX₆₀RFX₆₃GSGSGTDFTLTISX₇₇X₇₈QX₈₀EDX₈₃AX₈₅YFCHQY NTYPLTFGX₁₀₀GTK X₁₀₄EX₁₀₆X₁₀₇ (SEQ ID NO: 76), where X₃ may be Q or V, X₈ may be P or Q, X₉ may be K or S, X₁₀ may be F or S, X₁₁ may be L or M, X₁₃ may be A or T, X₂₀ may be S or T, X₂₁ may be I or V, X₄₂ may be K or Q, X₄₃ may be A or S, X₆₀ may be D or S, X₆₃ may be S or T, X₇₇ may be N or S, X₇₈ may be L or V, X₈₀ may be P or S, X₈₃ may be F or L, X₈₅ may be E or T, X₁₀₀ may be A or G, X₁₀₄ may be L or V, X₁₀₆ may be I or L, and X₁₀₇ may be K or N.

In the present application, the light chain variable region of the antigen binding protein may comprise an amino acid sequence shown in either SEQ ID NO: 31 or 47.

In the present application, the antigen binding protein may comprise a light chain constant region, and the light chain constant region may comprise a constant region derived from Igκ or a constant region derived from Igλ.

For example, the light chain constant region may comprise a constant region derived from Igκ.

For example, the light chain constant region of the antigen binding protein comprises an amino acid sequence shown in SEQ ID NO: 57.

In the present application, the antigen binding protein may comprise HCDR1-3 and LCDR1-3. For example, the HCDR1 of the antigen binding protein may comprise an amino acid sequence shown in SEQ ID NO: 41; the HCDR2 of the antigen binding protein may comprise an amino acid sequence shown in SEQ ID NO: 43; the HCDR3 of the antigen binding protein may comprise an amino acid sequence shown in SEQ ID NO: 45; the LCDR1 of the antigen binding protein may comprise an amino acid sequence shown in SEQ ID NO: 33; the LCDR2 of the antigen binding protein may comprise an amino acid sequence shown in SEQ ID NO: 35; and the LCDR3 of the antigen binding protein may comprise an amino acid sequence shown in SEQ ID NO: 37. For example, the antigen binding protein may comprise an antibody 47A1 or JYB1931A13 or an antigen binding fragment having the same HCDR3 (for example, having the same HCDR1-3) and LCDR3 (for example, having the same LCDR1-3).

In the present application, the antigen binding protein may comprise a heavy chain variable region and a light chain variable region. The heavy chain variable region of the antigen binding protein may comprise HCDR1-3 and H-FR1-4. The light chain variable region of the antigen binding protein may comprise LCDR1-3 and L-FR1-4. For example, the HCDR1 may comprise an amino acid sequence shown in SEQ ID NO: 41; the HCDR2 may comprise an amino acid sequence shown in SEQ ID NO: 43; the HCDR3 may comprise an amino acid sequence shown in SEQ ID NO: 45; the LCDR1 may comprise an amino acid sequence shown in SEQ ID NO: 33; the LCDR2 may comprise an amino acid sequence shown in SEQ ID NO: 35; and the LCDR3 may comprise an amino acid sequence shown in SEQ ID NO: 37. For example, the H-FR1 may comprise an amino acid sequence shown in SEQ ID NO: 40; the H-FR2 may comprise an amino acid sequence shown in SEQ ID NO: 42; the H-FR3 may comprise an amino acid sequence shown in SEQ ID NO: 44; the H-FR4 may comprise an amino acid sequence shown in SEQ ID NO: 46; the L-FR1 may comprise an amino acid sequence shown in SEQ ID NO: 32; the L-FR2 may comprise an amino acid sequence shown in SEQ ID NO: 34; the L-FR3 may comprise an amino acid sequence shown in SEQ ID NO: 36; and the L-FR4 may comprise an amino acid sequence shown in SEQ ID NO: 38. For example, the heavy chain variable region of the antigen binding protein may comprise an amino acid sequence shown in SEQ ID NO: 39. For example, the antigen binding protein may comprise an antibody 47A1 or an antigen binding protein having the same heavy chain variable region. For example, the light chain variable region of the antigen binding protein may comprise an amino acid sequence shown in SEQ ID NO: 31. For example, the antigen binding protein may comprise an antibody 47A1 or an antigen binding protein having the same light chain variable region.

In the present application, the antigen binding protein may comprise a heavy chain variable region and a light chain variable region, and the heavy chain variable region may comprise HCDR1-3 and H-FR1-4. The light chain variable region may comprise LCDR1-3 and L-FR1-4. For example, the HCDR1 may comprise an amino acid sequence shown in SEQ ID NO: 41; the HCDR2 may comprise an amino acid sequence shown in SEQ ID NO: 43; the HCDR3 may comprise an amino acid sequence shown in SEQ ID NO: 45; the LCDR1 may comprise an amino acid sequence shown in SEQ ID NO: 33; the LCDR2 may comprise an amino acid sequence shown in SEQ ID NO: 35; and the LCDR3 may comprise an amino acid sequence shown in SEQ ID NO: 37. For example, the H-FR1 may comprise an amino acid sequence shown in SEQ ID NO: 53; the H-FR2 may comprise an amino acid sequence shown in SEQ ID NO: 54; the H-FR3 may comprise an amino acid sequence shown in SEQ ID NO: 55; the H-FR4 may comprise an amino acid sequence shown in SEQ ID NO: 30; the L-FR1 may comprise an amino acid sequence shown in SEQ ID NO: 48; the L-FR2 may comprise an amino acid sequence shown in SEQ ID NO: 49; the L-FR3 may comprise an amino acid sequence shown in SEQ ID NO: 50; and the L-FR4 may comprise an amino acid sequence shown in SEQ ID NO: 51. For example, the heavy chain variable region of the antigen binding protein may comprise an amino acid sequence shown in SEQ ID NO: 52. For example, the antigen binding protein may comprise an antibody JYB1931A13 or an antigen binding protein having the same heavy chain variable region. For example, the light chain variable region of the antigen binding protein may comprise an amino acid sequence shown in SEQ ID NO: 47. For example, the antigen binding protein may comprise an antibody JYB1931A13 or an antigen binding protein having the same light chain variable region.

In the present application, the isolated antigen binding protein may further compete with a reference antibody to bind to the human MASP-2 protein, the reference antibody may comprise a heavy chain variable region VH, and the VH may comprise at least one, two, or three of HCDR1, HCDR2, and HCDR3.

In the present application, the HCDR3 of the reference antibody may comprise an amino acid sequence shown in SEQ ID NO: 19. For example, a sequence of the HCDR3 of the reference antibody may be defined according to the Chothia coding system.

In the present application, the HCDR2 of the reference antibody may comprise an amino acid sequence shown in SEQ ID NO: 17. For example, a sequence of the HCDR2 of the reference antibody may be defined according to the Chothia coding system.

In the present application, the HCDR1 of the reference antibody may comprise an amino acid sequence shown in SEQ ID NO: 15. For example, a sequence of the HCDR1 of the reference antibody may be defined according to the Chothia coding system.

For example, the HCDR1 of the reference antibody may comprise an amino acid sequence shown in SEQ ID NO: 15; the HCDR2 may comprise an amino acid sequence shown in SEQ ID NO: 17; and the HCDR3 may comprise an amino acid sequence shown in SEQ ID NO: 19. For example, the reference antibody may comprise an antibody 50A6 or JYB1931A63 or an antigen binding protein having the same HCDR3 (for example, having the same HCDR1-3).

In the present application, the reference antibody may comprise a heavy chain variable region, and the heavy chain variable region may comprise an amino acid sequence shown in SEQ ID NO: 67.

EVQLVESGGGLVQPGX₁₆SLX₁₉LSCAASGFTFNDYNMAWVRQX₄₀PX₄₂KGLE WVATILFDGSRTYYRDSVKGRFTISRX₇₃NAKX₇₇X₇₈LYLQMX₈₄SERX₈₈EDTAX₉₃YY CSTESPYYSEGYYQGYFDYWGQGX₁₁₉X₁₂₀VTVSS (SEQ ID NO: 67), where X₁₆ may be G or R, X₁₉ may be R or S, X₄₀ may be A or T, X₄₂ may be G or K, X₇₃ may be D or E, X₇₇ may be N or S, X₇₈ may be S or T, X₈₄ may be D or N, X₈₈ may be A or S, X₉₃ may be T or V, X₁₁₉ may be T or V, and X₁₂₀ may be L or M.

In the present application, the heavy chain variable region of the reference antibody may comprise an amino acid sequence shown in any of SEQ ID NO: 13 and SEQ ID NO: 26.

In the present application, the reference antibody may comprise a heavy chain constant region, and the heavy chain constant region may comprise a constant region derived from IgG or a constant region derived from IgY.

For example, the heavy chain constant region of the reference antibody may comprise an amino acid sequence shown in SEQ ID NO: 56.

In the present application, the reference antibody may comprise a light chain variable region VL, and the VL may comprise LCDR1, LCDR2, and LCDR3.

In the present application, the LCDR3 of the reference antibody may comprise an amino acid sequence shown in SEQ ID NO: 11. For example, a sequence of the LCDR3 of the reference antibody may be defined according to the Chothia coding system.

In the present application, the LCDR2 of the reference antibody may comprise an amino acid sequence shown in SEQ ID NO: 9. For example, a sequence of the LCDR2 of the reference antibody may be defined according to the Chothia coding system.

In the present application, the LCDR1 of the reference antibody may comprise an amino acid sequence shown in SEQ ID NO: 7. For example, a sequence of the LCDR1 of the reference antibody may be defined according to the Chothia coding system.

For example, the LCDR1 of the reference antibody described in the present application may comprise an amino acid sequence shown in SEQ ID NO: 7; the LCDR2 may comprise an amino acid sequence shown in SEQ ID NO: 9; and the LCDR3 may comprise an amino acid sequence shown in SEQ ID NO: 11. For example, the reference antibody may comprise an antibody 50A6 or JYB1931A63 or an antigen binding protein having the same LCDR3 (for example, having the same LCDR1-3).

In the present application, the reference antibody may comprise a light chain variable region, and the light chain variable region may comprise an amino acid sequence shown in SEQ ID NO: 66.

X₁IVLTQSPATLSX₁₃X₁₄PGEX₁₈X₁₉X₂₀LSCRASQSVSTSIHWYQQX₃₉X₄₀X₄₁X₄₂X ₄₃PRLLIKYASRSISGIPX₆₀RFSGSGSGTDFTLX₇₄IX₇₆X₇₇X₇₈EX₈₀EDFX₈₄X₈₅YYCQQS YSSLYTFGX₁₀₀GTKLE X₁₀₆X₁₀₇ (SEQ ID NO: 66), where X₁ may be E or N, X₁₃ may be L or V, X₁₄ may be S or T, X₁₈ may be R or S, X₁₉ may be A or V, X₂₀ may be S or T, X₃₉ may be K or R, X₄₀ may be P or S, X₄₁ may be G or N, X₄₂ may be E or Q, X₄₃ may be A or S, X₆₀ may be A or S, X₇₄ may be S or T, X₇₆ may be N or S, X₇₇ may be R or S, X₇₈ may be L or V, X₈₀ may be P or S, X₈₄ may be A or S, X₈₅ may be I or V, X₁₀₀ may be A or Q, X₁₀₆ may be I or L, and X₁₀₇ may be K or R.

In the present application, the light chain variable region of the reference antibody may comprise an amino acid sequence shown in any of SEQ ID NO: 5 and SEQ ID NO: 21.

In the present application, the reference antibody may comprise HCDR1-3 and LCDR1-3. For example, the HCDR1 may comprise an amino acid sequence shown in SEQ ID NO: 15; the HCDR2 may comprise an amino acid sequence shown in SEQ ID NO: 17; the HCDR3 may comprise an amino acid sequence shown in SEQ ID NO: 19; the LCDR1 may comprise an amino acid sequence shown in SEQ ID NO: 7; the LCDR2 may comprise an amino acid sequence shown in SEQ ID NO: 9; and the LCDR3 may comprise an amino acid sequence shown in SEQ ID NO: 11. For example, the reference antibody may comprise an antibody 50A6 or JYB1931A63 or an antigen binding protein having the same HCDR3 (for example, having the same HCDR1-3) and HCDR3 (for example, having the same LCDR1-3).

In the present application, the isolated antigen binding protein may further compete with a reference antibody to bind to the human MASP-2 protein, the reference antibody may comprise a heavy chain variable region VH, and the VH may comprise at least one, two, or three of HCDR1, HCDR2, and HCDR3.

In the present application, the HCDR3 of the reference antibody may comprise an amino acid sequence shown in SEQ ID NO: 45. For example, a sequence of the HCDR3 of the reference antibody may be defined according to the Chothia coding system.

In the present application, the HCDR2 of the reference antibody may comprise an amino acid sequence shown in SEQ ID NO: 43. For example, a sequence of the HCDR2 of the reference antibody may be defined according to the Chothia coding system.

In the present application, the HCDR1 of the reference antibody may comprise an amino acid sequence shown in SEQ ID NO: 41. For example, a sequence of the HCDR1 of the reference antibody may be defined according to the Chothia coding system.

For example, the HCDR1 of the reference antibody may comprise an amino acid sequence shown in SEQ ID NO: 41; the HCDR2 may comprise an amino acid sequence shown in SEQ ID NO: 43; and the HCDR3 may comprise an amino acid sequence shown in SEQ ID NO: 45. For example, the reference antibody may comprise an antibody 47A1 or JYB1931A13 or an antigen binding protein having the same HCDR3 (for example, having the same HCDR1-3).

In the present application, the reference antibody may comprise a heavy chain variable region, and the heavy chain variable region may comprise an amino acid sequence shown in SEQ ID NO: 77.
X₁VQLQESGPGLVKPSQX₁₇LSLTCTVX₂₅GYSITSDYAWNWIRQX₄₁PGX₄₄X₄₅LE W

X₄₉GYISYSGRTSYNPSLKSRX₆₈X₆₉IX₇₁RX₇₃TSX₇₆NQFX₈₀LX₈₂LX₈₄SVTX₈₈X₈₉DTA X₉₃YX₉₅CARYWGDYWGQGT X₁₀₉X₁₁₀TVSS (SEQ ID NO: 77), where X₁ may be D or Q, X₁₇ may be S or T, X₂₅ may be S or T, X₄₁ may be F or P, X₄₄ may be K or N, X₄₅ may be G or K, X₄₉ may be I or M, X₆₈ may be I or V, X₆₉ may be S or T, X₇₁ may be S or T, X₇₃ may be D or N, X₇₆ may be K or T, X₈₀ may be F or S, X₈₂ may be K or Q, X₈₄ may be N or S, X₈₈ may be A or T, X₈₉ may be A or E, X₉₃ may be T or V, X₉₅ may be F or Y, X₁₀₉ may be L or T, and X₁₁₀ may be L or V.

In the present application, the heavy chain variable region of the reference antibody may comprise an amino acid sequence shown in any of SEQ ID NO: 39 and SEQ ID NO: 52.

In the present application, the reference antibody may comprise a heavy chain constant region, and the heavy chain constant region may comprise a constant region derived from IgG or a constant region derived from IgY.

For example, the heavy chain constant region of the reference antibody may comprise an amino acid sequence shown in SEQ ID NO: 56.

In the present application, the reference antibody may comprise a light chain variable region VL, and the VL may comprise LCDR1, LCDR2, and LCDR3.

In the present application, the LCDR3 of the reference antibody may comprise an amino acid sequence shown in SEQ ID NO: 37. For example, a sequence of the LCDR3 of the reference antibody may be defined according to the Chothia coding system.

In the present application, the LCDR2 of the reference antibody may comprise an amino acid sequence shown in SEQ ID NO: 35. For example, a sequence of the LCDR2 of the reference antibody may be defined according to the Chothia coding system.

In the present application, the LCDR1 of the reference antibody may comprise an amino acid sequence shown in SEQ ID NO: 33. For example, a sequence of the LCDR1 of the reference antibody may be defined according to the Chothia coding system.

For example, the LCDR1 of the reference antibody described in the present application may comprise an amino acid sequence shown in SEQ ID NO: 33; the LCDR2 may comprise an amino acid sequence shown in SEQ ID NO: 35; and the LCDR3 may comprise an amino acid sequence shown in SEQ ID NO: 37. For example, the reference antibody may comprise an antibody 47A1 or JYB1931A13 or an antigen binding protein having the same LCDR3 (for example, having the same LCDR1-3).

In the present application, the reference antibody may comprise a light chain variable region, and the light chain variable region may comprise an amino acid sequence shown in SEQ ID NO: 76.

DIX₃MTQSX₈X₉X₁₀X₁₁SX₁₃SVGDRVX₂₀X₂₁TCKASQNVGSNVAWFQQKPGX₄₂X ₄₃PKPLIYSASYRYSGVPX₆₀RFX₆₃GSGSGTDFTLTISX₇₇X₇₈QX₈₀EDX₈₃AX₈₅YFCHQY NTYPLTFGX₁₀₀GTKX₁₀₄EX₁₀₆X₁₀₇ (SEQ ID NO: 76), where X₃ may be Q or V, X₈ may be P or Q, X₉ may be K or S, X₁₀ may be F or S, X₁₁ may be L or M, X₁₃ may be A or T, X₂₀ may be S or T, X₂₁ may be I or V, X₄₂ may be K or Q, X₄₃ may be A or S, X₆₀ may be D or S, X₆₃ may be S or T, X₇₇ may be N or S, X₇₈ may be L or V, X₈₀ may be P or S, X₈₃ may be F or L, X₈₅ may be E or T, X₁₀₀ may be A or G, X₁₀₄ may be L or V, X₁₀₆ may be I or L, and X₁₀₇ may be K or N.

In the present application, the light chain variable region of the reference antibody may comprise an amino acid sequence shown in either SEQ ID NO: 31 or 47.

In the present application, the reference antibody may comprise HCDR1-3 and LCDR1-3. For example, the HCDR1 may comprise an amino acid sequence shown in SEQ ID NO: 41; the HCDR2 may comprise an amino acid sequence shown in SEQ ID NO: 43; the HCDR3 may comprise an amino acid sequence shown in SEQ ID NO: 45; the LCDR1 may comprise an amino acid sequence shown in SEQ ID NO: 33; the LCDR2 may comprise an amino acid sequence shown in SEQ ID NO: 35; and the LCDR3 may comprise an amino acid sequence shown in SEQ ID NO: 37. For example, the reference antibody may comprise an antibody 47A1 or JYB1931A13 or an antigen binding protein having the same HCDR3 (for example, having the same HCDR1-3) and HCDR3 (for example, having the same LCDR1-3).

In the present application, the reference antibody may comprise a heavy chain variable region and a light chain variable region. For example, the heavy chain variable region of the reference antibody may comprise an amino acid sequence shown in SEQ ID NO: 13. For example, the reference antibody may comprise an antibody 50A6 or an antigen binding protein having the same heavy chain variable region. For example, the light chain variable region of the reference antibody may comprise an amino acid sequence shown in SEQ ID NO: 5. For example, the reference antibody may comprise an antibody 50A6 or an antigen binding protein having the same light chain variable region. For example, the reference antibody may comprise an antibody 50A6 or an antigen binding protein having the same heavy chain variable region and light chain variable region.

In the present application, the reference antibody may comprise a heavy chain variable region and a light chain variable region. For example, the heavy chain variable region of the reference antibody may comprise an amino acid sequence shown in SEQ ID NO: 26. For example, the reference antibody may comprise an antibody JYB1931A63 or an antigen binding protein having the same heavy chain variable region. For example, the light chain variable region of the reference antibody may comprise an amino acid sequence shown in SEQ ID NO: 21. For example, the reference antibody may comprise an antibody JYB 1931A63 or an antigen binding protein having the same light chain variable region. For example, the reference antibody may comprise an antibody JYB 1931A63 or an antigen binding protein having the same heavy chain variable region and light chain variable region.

In the present application, the reference antibody may comprise a heavy chain variable region and a light chain variable region. For example, the heavy chain variable region of the reference antibody may comprise an amino acid sequence shown in SEQ ID NO: 39. For example, the reference antibody may comprise an antibody 47A1 or an antigen binding protein having the same heavy chain variable region. For example, the light chain variable region of the reference antibody may comprise an amino acid sequence shown in SEQ ID NO: 31. For example, the reference antibody may comprise an antibody 47A1 or an antigen binding protein having the same light chain variable region. For example, the reference antibody may comprise an antibody 47A1 or an antigen binding protein having the same heavy chain variable region and light chain variable region.

In the present application, the reference antibody may comprise a heavy chain variable region and a light chain variable region. For example, the heavy chain variable region of the reference antibody may comprise an amino acid sequence shown in SEQ ID NO: 52. For example, the reference antibody may comprise an antibody JYB1931A13 or an antigen binding protein having the same heavy chain variable region. For example, the light chain variable region of the reference antibody may comprise an amino acid sequence shown in SEQ ID NO: 47. For example, the reference antibody may comprise an antibody JYB 1931A13 or an antigen binding protein having the same light chain variable region. For example, the reference antibody may comprise an antibody JYB1931A13 or an antigen binding protein having the same heavy chain variable region and light chain variable region.

### Polypeptide and immunoconjugate

In another aspect, the present application provides one or more polypeptides, which may include the isolated antigen binding protein of the present application. For example, the polypeptide may include a fusion protein. For example, the polypeptide may include a multi-specific antibody (such as a bispecific antibody).

In another aspect, the present application provides one or more immunoconjugates, which may include the isolated antigen binding protein of the present application. In some embodiments, the immunoconjugate may further include pharmaceutically acceptable therapeutic agents, markers, and/or detection agents.

### Nucleic acid, vector, and cell

In another aspect, the present application further provides one or more isolated nucleic acid molecules, which can encode the isolated antigen binding protein of the present application. For example, each of the one or more nucleic acid molecules can encode the entire antigen binding protein or a portion thereof (such as one or more of HCDR1-3 and the heavy chain variable region).

For example, when the nucleic acid molecule encodes a portion of the antigen binding protein, products encoded by the nucleic acid molecule can combine to form the functional (for example, capable of binding to MASP-2) isolated antigen binding protein of the present application.

The nucleic acid molecule described in the present application can be isolated. For example, the nucleic acid molecule can be produced or synthesized by the following method: (i) in vitro amplification, such as polymerase chain reaction (PCR) amplification, (ii) cloning and recombination, (iii) purification, such as enzyme digestion and gel electrophoresis fractionation, or (iv) synthesis, such as chemical synthesis. For example, the isolated nucleic acid may be a nucleic acid molecule prepared by recombinant DNA technology.

In the present application, the nucleic acid encoding the isolated antigen binding protein can be prepared by multiple methods known in the art, including but not limited to use of reverse transcription PCR and PCR to obtain nucleic acid molecules of the isolated antigen binding protein described in the present application.

In another aspect, the present application provides one or more vectors, including one or more nucleic acid molecules described in the present application. Each vector may include one or more of the nucleic acid molecules. Moreover, the vector may further include other genes, such as marker genes allowing the vector to be selected in appropriate host cells and under appropriate conditions. In addition, the vector may further include expression control elements allowing a coding region to be correctly expressed in an appropriate host. Such control elements are well-known to those skilled in the art, for example, may include promoters, ribosome binding sites, enhancers, other control elements adjusting gene transcription or mRNA translation, and the like. In some embodiments, the expression control sequence is a adjustable element. A specific structure of the expression control sequence may vary according to the function of a species or cell type, but generally includes 5' non-transcriptional sequences and 5' and 3' non-translational sequences involved in transcription and translation initiation, respectively, such as a TATA box, a capped sequence, and a CAAT sequence. For example, the 5' non-transcriptional expression control sequence may include a promoter region, which may include a promoter sequence used for transcriptional control functional linkage of nucleic acids. The expression control sequence may further include an enhancer sequence or an upstream activator sequence. In the present application, an appropriate promoter may include, for example, promoters for SP6, T3, and T7 polymerase, a human U6RNA promoter, a CMV promoter and an artificial hybrid promoter thereof (such as CMV), where a portion of the promoter can fuse with a portion of other cell protein (such as human GAPDH and glyceraldehyde-3-phosphate dehydrogenase) gene promoters, which may or may not include additional introns. The one or more nucleic acid molecules described in the present application may be operably connected to the expression control element.

The vector may include, for example, plasmids, cosmids, viruses, bacteriophages, or other vectors commonly used in genetic engineering. For example, the vector may be an expression vector. For example, the vector may be a viral vector. The viral vector may be administered to a patient (in vivo) directly or indirectly, for example, by treating cells with a virus in vitro and then administering the treated cells to a patient (in vitro). The virus vector technology is well known in the art and has been described by Sambrook et al. (2001, Molecular Cloning: A Laboratory Manual, Cold Spring Harbor Laboratory, New York) and in other virology and molecular biology manuals. Conventional virus-based systems may include retroviral vectors, lentiviral vectors, adenoviral vectors, adeno-associated virus vectors, and herpes simplex virus vectors for gene transfer. In some cases, genes may be transferred and integrated into host genomes by retroviruses, lentiviruses, and adeno-associated viruses to express the inserted genes for a long term. The lentiviral vectors are retroviral vectors that can transduce or infect non-dividing cells and typically generate high viral titers. The lentiviral vector may include a long terminal repeat sequence 5' LTR and truncated 3' LTR, RRE, rev response element (cPPT), central termination sequence (CTS) and/or a translated regulatory element (WPRE). The vector described in the present application can be introduced into cells.

In another aspect, the present application provides a cell. The cell may include the isolated antigen binding protein, the polypeptide, the immunoconjugate, the one or more nucleic acid molecules, and/or the one or more vectors described in the present application. For example, each type of cells or each cell may include the one or more nucleic acid molecules or vectors described in the present application. For example, each type of cells or each cell may include a plurality of (such as 2 or more) or multiple types of (such as 2 or more types of) nucleic acid molecules or vectors described in the present application. For example, the vector described in the present application can be introduced into the host cells, such as prokaryotic cells (such as bacterial cells), CHO cells, NS/0 cells, HEK293T cells, 293F cells, or HEK293A cells, or other eukaryotic cells, such as cells from plants, fungi, or yeast cells. The vector described in the present application can be introduced into the host cells by methods known in the art, such as electroporation, lipofectine transfection, and lipofectamine transfection. For example, the cells may include yeast cells. For example, the cells may include Escherichia coli cells. For example, the cells may include mammalian cells. For example, the cells may include immune cells.

The cells may include immune cells. In some cases, the cells may include immune cells. For example, the cells may include T cells, B cells, natural killer (NK) cells, macrophages, NKT cells, monocytes, dendritic cells, granulocytes, lymphocytes, white blood cells, and/or peripheral blood mononuclear cells.

### Pharmaceutical composition

In another aspect, the present application provides a pharmaceutical composition. The pharmaceutical composition may include the isolated antigen binding protein, the polypeptide, the immunoconjugate, the isolated nucleic acid molecule, the vector, and the cell of the present application, and/or pharmaceutically acceptable adjuvants and/or excipients. In the present application, the pharmaceutically acceptable adjuvants may include buffering agents, antioxidants, preservatives, low molecular weight polypeptides, proteins, hydrophilic polymers, amino acids, saccharides, chelating agents, counter ions, metal complexes, and/or non-ionic surfactants. Unless incompatible with the cells described in the present application, any conventional medium or reagent can be considered for use in the pharmaceutical composition of the present application. In the present application, the pharmaceutically acceptable excipients may include additives in the pharmaceutical formulation other than the main drug, or may be referred to as accessories. For example, the excipients may include adhesives, fillers, disintegrants, and lubricants in tablets. For example, the excipients may include alcohol, vinegar, medicinal juice, and the like in traditional Chinese medicine pills. For example, the excipients may include matrix portions in semi-solid formulation ointments or creams. For example, the excipients may include preservatives, antioxidants, flavoring agents, aromatics, cosolvents, emulsifiers, solubilizers, osmotic pressure regulators, and colorants in liquid formulations.

### Kit, use and method

In another aspect, the present application provides a method for detecting or measuring MASP-2, the method may include use of the isolated antigen binding protein or the polypeptide.

In the present application, the method may include in vitro methods, in vitro methods, and non-diagnostic or non-therapeutic methods.

For example, the method may include a method for detecting the presence and/or content of MASP-2 for non-diagnostic purposes, which may include the following steps:
1) Contact a sample with the antigen binding protein of the present application; and
2) Detect the presence and/or content of the antigen binding protein bound to the sample to determine the presence and/or expression level of MASP-2 in the sample obtained from a subject.

In another aspect, the present application provides a MASP-2 kit, which may include use of the isolated antigen binding protein or the polypeptide.

In the present application, the kit may further include instructions for use, which document methods for detecting the presence and/or content of MASP-2. For example, the methods may include in vitro methods, in vitro methods, and non-diagnostic or non-therapeutic methods.

In another aspect, the present application provides a use of the isolated antigen binding protein or the polypeptide in preparation of a kit for methods for detecting the presence and/or content of MASP-2. For example, the methods may include in vitro methods, ex vivomethods, and non-diagnostic or non-therapeutic methods.

In another aspect, the present application provides a use of the isolated antigen binding protein, the polypeptide, the immunoconjugate, the isolated nucleic acid molecule, the vector, and the pharmaceutical composition for preventing, alleviating, and/or treating diseases or conditions.

In another aspect, the present application provides a use of the isolated antigen binding protein, the polypeptide, the immunoconjugate, the isolated nucleic acid molecule, the vector, the cell, and/or the pharmaceutical composition in preparation of medicaments for preventing, alleviating, and/or treating diseases or conditions.

In another aspect, the present application provides a method for preventing and/or treating diseases or conditions, which includes administering, to subjects in need, the isolated antigen binding protein, the isolated nucleic acid molecule, the vector, the cell, and the pharmaceutical composition.

The pharmaceutical composition, pharmaceutical combination and method described in the present application can be used with other types of cancer therapies, such as chemotherapy, surgery, radiation, and gene therapy. The pharmaceutical composition and method described in the present application can be used for other disease symptoms that depend on immune response, such as inflammation, immune diseases, and infectious diseases.

In the present application, the subjects may include humans or non-human animals. For example, the non-human animals may be selected from the following group: monkeys, chickens, geese, cats, dogs, mice, and rats. In addition, the non-human animals may further include any animal species other than humans, such as livestock, rodents, primates, domesticated animals, or poultry animals. The humans may be Caucasian, African, Asian, Semitic, or other races, or a hybrid of various races. For example, the humans may be the elderly, adults, teenagers, children, or infants.

An effective amount in humans can be inferred based on an effective amount in experimental animals. For example, Freireich et al. described correlations between doses for animals and humans (based on milligrams per square meter of body surface) (Freireich et al., Cancer Chemother. Rep. 50, 219 (1966)). The body surface area can be approximately determined from the patient's height and weight. For example, see Scientific Tables, Geigy Pharmaceuticals, Ardsley, N.Y., 537 (1970).

Without being limited by any theory, the following examples are only intended to illustrate the fusion protein, preparation method, and use of the present application, and are not intended to limit the scope of the present invention.

### Examples

### Example 1 Preparation of antigen

A human MASP-2 protein (000187) included six functional domains. In this implementation, domain 4 (CPP1), domain 5 (CPP2), and domain 6 (SP) were selected for recombinant expression in Escherichia coli. The expressed protein existed in a form of an inclusion body. In order to obtain an active protein, the inclusion body was renaturated and then purified through C terminal His of a protein fragment. The obtained recombinant human MASP-2 proteins were hMASP2-D456 (an amino acid sequence of which was as shown in SEQ ID NO: 1) and hMASP2A-D456 (an amino acid sequence of which was as shown in SEQ ID NO: 2), respectively.

The same preparation method was used to prepare cynomolgus monkey (A0A2K5UJY0|, cMASP2-D456, an amino acid sequence of which was as shown in SEQ ID NO: 4) and mouse (Q91WP0, mMASP2-D456, an amino acid sequence of which was as shown in SEQ ID NO: 3) MASP-2.

### Example 2 Preparation of rat/mouse anti-human MASP-2 monoclonal antibodies

### 2.1 Animal immunization

At initial immunization, the hMASP2A-D456 and PADRE were emulsified in a 1:1 ratio by using a complete Freund's adjuvant, and then intraperitoneally injected into 6-8 week old female SD rats and intraperitoneally and subcutaneously injected at multiple sites into 6-8 week old female Balb/c mice, 50 µg hMASP2A-D456 per rat (mouse). Afterwards, booster immunization was performed at an interval of two to three weeks, with each rat injected intraperitoneally with 50 µg antigen plus Freund's incomplete adjuvant, and each mouse injected intraperitoneally and subcutaneously at multiple sites with 50 µg antigen plus Freund's incomplete adjuvant. A total of 3 immunizations were performed, animal tail blood was collected two weeks after the last immunization, and a serum anti-human MASP-2 antibody titer was measured. Animals to be fused were injected intraperitoneally with 50 µg protein for shock immunization, not added with the Freund's adjuvant, and spleen cells were taken on the third day.

### 2.2 Spleen cell fusion

The rats/mice were dissected after euthanasia, spleens were obtained and ground, cells were collected by centrifuging at 1500 rpm for 5 minutes, the cells were suspended with 5-10 milliliters of red blood cell lysate, the suspension was placed at 4°C for 10 minutes, the reaction was terminated with DMEM + 10% FBS, and cells were counted. The cells were suspended with 40 ml of DMEM after centrifugation, the suspension was stood for 2-3 minutes, and then the supernatant was transferred to another 50 ml centrifuge tube. SP2/0 cells were collected and mixed with the spleen cells in a ratio of 1:2, the mixed cells were centrifuged, the supernatant was fully extracted, the mixed cells were precipitated by patting and washed twice with DMEM, and PEG fusion was performed according to a conventional method. After fusion, the cells were washed with a DMEM medium and resuspended in a DMEM + 10% FBS + 1 × HAT screening medium. The fused cells were added to a 96-well cell culture plate and cultured in a 37°C, 75% humidity, and 5% CO₂ incubator for 9-10 days.

### 2.3 Screening of rat/mouse monoclonal antibodies

### (1) ELISA identification on function of binding hybridoma monoclonal culture supernatant to hMASP2-D456

The hMASP2-D456 was diluted to 1.0 µg/mL with a carbonate buffer solution, added to a high binding transparent polystyrene 96-well plate (Nunc) with 100 µL/well, and wrapped overnight at 4°C. The next day, the ELISA plate was washed twice on an automatic plate washer with a washing buffer (PBS + 0.05% Tween 20 (sigma)). 300 µL of blocking buffer (PBS + 0.05% Tween 20 (sigma) + 1% BSA) was added to each well, and the ELISA plate was sealed at room temperature for 1 hour. Then the ELISA plate was washed twice with the washing buffer on the automatic plate washer, 1000 µL of supernatant of hybridoma was transferred to each well of the ELISA plate, incubation was performed at room temperature for 1 hour, and then the plate was washed 3 times according to the above method. 100 µL of goat anti-mouse HRP (Sigma, article number: M4280) or rabbit anti-rat Fc-HRP (Sigma, article number: A5795) diluted at 1:5000 in the blocking buffer was added to each well. Incubation was performed at room temperature for 1 hour, and then the plate was washed 3 times according to the above method. TMB substrate solution was added with 100 µL/well, then 50 µL of 1.0 M hydrochloric acid termination solution was added to each well to terminate the reaction, and the plate was read at 450 nm on a Thermo Multiscan FC. By combining experiments, hybridoma cloned rats 50A6 and mice 47A1 were screened out. A hybridoma monoclonal antibody was prepared by serum-free culture and a conventional antibody purification method to confirm its function.

### Example 3 Identification of rat/mouse anti-human MASP-2 monoclonal antibodies

### 3.1 ELISA identification on function of binding rat/mouse anti-human MASP-2 monoclonal antibodies to human MASP-2

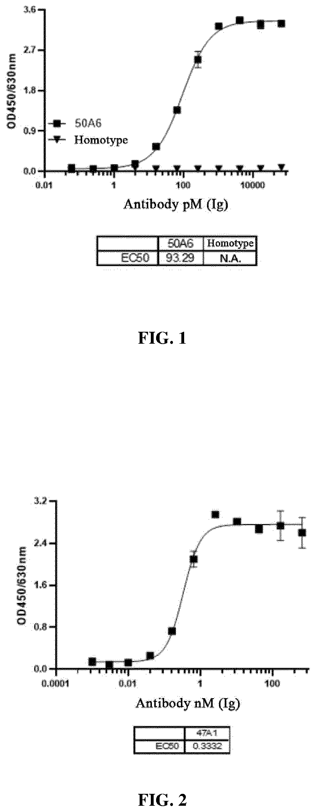

The hMASP2-D456 was diluted to 1.0 µg/mL and 0.5 µg/mL with a carbonate buffer solution (CBS), each added to a high binding transparent polystyrene 96-well plate (Nunc) with 100 µL/well and wrapped overnight at 4°C. The next day, the ELISA plate was washed twice on an automatic plate washer with a washing buffer (PBS + 0.05% Tween 20 (sigma)). 300 µL of blocking buffer (PBS + 0.05% Tween 20 (sigma) + 1% BSA) was added to each well, and the ELISA plate was sealed at room temperature for 1 hour. Then the ELISA plate was washed twice with the washing buffer on the automatic plate washer, and the monoclonal antibody was diluted with the blocking buffer to 8 gradients. The monoclonal antibody was sequentially added to each well of the ELISA plate and incubated at room temperature for 1 hour, and then the plate was washed 3 times according to the above method. 100 µL of goat anti-mouse HRP (Sigma, article number: M4280) or rabbit anti-rat Fc-HRP (Sigma, article number: A5795) diluted at 1:5000 in the blocking buffer was added to each well. Incubation was performed at room temperature for 1 hour, and then the plate was washed 3 times according to the above method. A TMB substrate solution was added with 100 µL/well, and then 50 µL of 1.0 M hydrochloric acid termination solution was added to each well to terminate the reaction. The plate was read at 450 nm on a Thermo Multiscan FC. Graphs were drawn with Graphpad and EC50 was calculated. As shown in FIG. 1, the 50A6 antibody can bind to human MASP-2. As shown in FIG. 2, the 47A1 antibody can bind to human MASP-2. Therefore, the rat/mouse anti-human MASP-2 monoclonal antibodies in the present application can bind to human MASP-2.

### 3.2 ELISA identification on functions of cross binding of rat/mouse anti-human MASP-2 monoclonal antibodies to mouse mMASP2-D456 and cynomolgus monkey cMASP2-D456

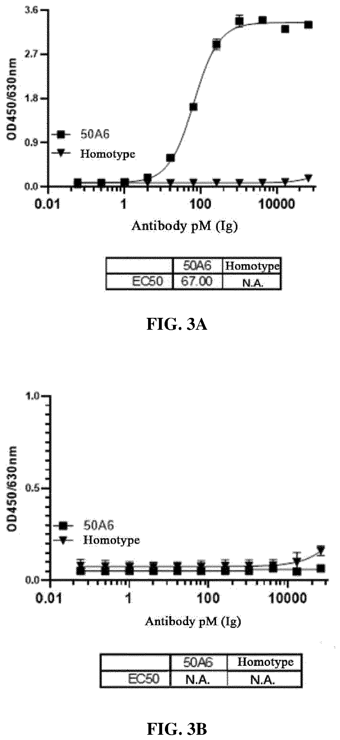

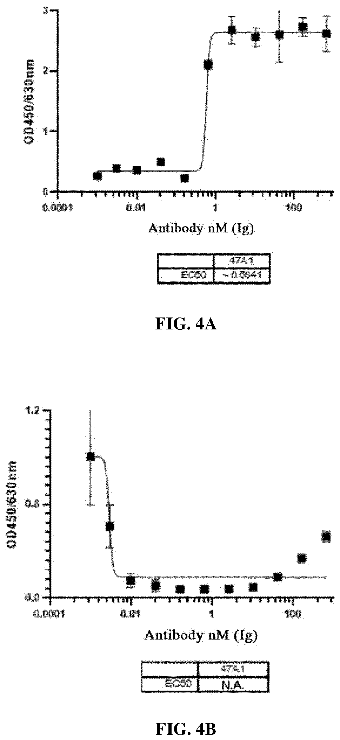

The mouse mMASP2-D456 and cynomolgus monkey cMASP2-D456 were diluted to 1.0 µg/mL and 0.5 µg/mL with a carbonate buffer solution, each added to a high binding transparent polystyrene 96-well plate (Nunc) with 100 µL/well and wrapped overnight at 4°C. The next day, the ELISA plate was washed twice on an automatic plate washer with a washing buffer (PBS + 0.05% Tween 20 (sigma)). 300 µL of blocking buffer (PBS + 0.05% Tween 20 (sigma) + 1% BSA) was added to each well, and the ELISA plate was sealed at room temperature for 1 hour. Then the ELISA plate was washed twice with the washing buffer on the automatic plate washer, and the monoclonal antibody 50A6 was diluted with the blocking buffer to 15.0 µg/mL at 11 gradients. The monoclonal antibody was sequentially added to each well of the ELISA plate and incubated at room temperature for 1 hour, and then the plate was washed 3 times according to the above method. 100 µL of goat anti-mouse HRP (Sigma, article number: M4280) or rabbit anti-rat Fc-HRP (Sigma, article number: A5795) diluted at 1:5000 with the blocking buffer was added to each well. Incubation was performed at room temperature for 1 hour, and then the plate was washed 3 times according to the above method. A TMB substrate solution was added with 100 µL/well, and then 50 µL of 1.0 M hydrochloric acid termination solution was added to each well to terminate the reaction. The plate was read at 450 nm on a Thermo Multiscan FC. Graphs were drawn with Graphpad and EC50 was calculated. The results of binding 50A6 to cynomolgus monkey cMASP2-D456 were shown in FIG. 3A, and the results of binding 50A6 to mouse mMASP2-D456 were shown in FIG. 3B. The results of binding 47A1 to cynomolgus monkey cMASP2-D456 were shown in FIG. 4A, and the results of binding 47A1 to mouse mMASP2-D456 were shown in FIG. 4B.

By identifying the functions of binding, blocking, and the like, 50A6 was selected as a rat derived candidate antibody and humanized, and 47A1 was selected as a mouse derived candidate antibody and humanized.

### Example 4 Humanization of hybridoma monoclonal antibody

4.1 Most homologous human germline sequences (data source: IMGT) were selected from the rat derived antibody 50A6 by sequence comparison as humanization design frameworks (light chain using GKV6-21 * 02 and IGKJ2 * 01 as frameworks; heavy chain using IGHV3-7 * 01 and IGH4 * 01 as frameworks); and the mouse derived antibody 47A1 used IGKV1-16 * 01, IGKJ4 * 01, IGHV4-30-4 * 01, and IGHJ4 * 01 as light and heavy chain frameworks, respectively. Light and heavy chain variable regions of the antibody were numbered by Chothia (see Chothia&Lesk, 1987) to define CDR regions of the antibody: CDRL1 (L24-L34, representing 24^{th}-34^{th} amino acids of VL), CDRL2 (L50-L56), CDRL3 (L89-L97), CDRH1 (H26-H32, representing 26^{th}-32^{th} amino acids of VH), CDRH2 (H52-H56), and CDRH3 (H95-H97). Humanized mutations were performed on the amino acids in the light and heavy chain variable regions of the antibody based on sequence comparison and structural information of the variable regions.

4.2 An expression vector was designed, genes were synthesized, a recombinant antibody was expressed and purified in mammalian cells, differences in activity and physical and chemical properties of the humanized antibody were compared, and 1-2 rounds of humanization optimization were performed.

4.3 The above germline antibody was used as a framework for CDR transplantation. First, variable region amino acid sequences of the following chimeric antibody were obtained: a light chain variable region sequence of 50A6 (its amino acid sequence was as shown in SEQ ID NO: 5); a heavy chain variable region sequence of 50A6 (its amino acid sequence was as shown in SEQ ID NO: 13);
a light chain variable region sequence of 47A1 (its amino acid sequence was as shown in SEQ ID NO: 31); and a heavy chain variable region sequence of 47A1 (its amino acid sequence was as shown in SEQ ID NO: 39).

Second, on the basis of the variable region amino acid sequences of the foregoing chimeric antibody, amino acid mutations were performed to obtain the following humanized optimized amino acid sequences: a light chain variable region sequence of JYB1931A63 (its amino acid sequence was as shown in SEQ ID NO: 21); a heavy chain variable region sequence of JYB 1931A63 (its amino acid sequence was shown in SEQ ID NO: 26);
a light chain variable region sequence of JYB1931A13 (its amino acid sequence was as shown in SEQ ID NO: 47); and a heavy chain variable region sequence of JYB1931A13 (its amino acid sequence was as shown in SEQ ID NO: 52).

### 4.4 Expression and purification of antibody

After the foregoing humanized optimized amino acid sequences were designed, codon optimization was performed on them respectively, and corresponding DNA gene fragments (Genscript) were synthesized. The synthesized gene fragments were amplified into an expression vector pcDNA3.4 (Life Technologies). After amplification and extraction of expression plasmids, double plasmids were co-transfected into ExpiCHO cells (ThermoFisher Scientific, A29133). Transient expression of the antibody was performed according to the supplier's ExpiCHO expression system method. The process was as follows: ExpiCHO cells were cultured in totally 25 mL of medium at 36.5°C and 8% carbon dioxide concentration to a density of 6×10⁶/mL and transfected with each 10 µg of light and heavy chain expression plasmids of the antibody by using an ExpiFectamine reagent; after one day of transfection, 150 µL of each sample and 4 mL of ExpiCHO enhancer and ExpiCHO accessories were added to the cultured cells, the culture cultured for 9 days, and a supernatant was obtained at 4°C and 3500 rpm. AmMagTM Protein A magnetic beads (Genscript, L00695) and the antibody expression supernatant were mixed, the cells were incubated at room temperature for 2 hours and washed twice with PBS to discard the supernatant, and an appropriate amount of eluting buffer protein G or A SefinoseTMElution buffer (Sangon, C600481) was added, followed by thorough mixing and static incubation on test tube rack for 5 minutes; and the magnetic beads were resuspended 2-3 times during incubation and repeatedly eluted twice, and then an appropriate amount of neutralization solution 1M Tris-HCl was immediately added to regulate the pH value to 7.5 (Sangon, B548124) for later use.

### Example 5: Anti-MASP-2 mouse/rat derived antibodies blocked a lectin pathway of a human complement system

5.1 Blocking of a signaling pathway of a human serum complement by the antibodies was detected by a WIESLAB^{®} complement system lectin pathway (Svar Life Science AB, article number: AS 1327) kit. Human serum and the antibodies 50A6 and 47A1 were diluted with three diluents: CP: 80 µl/4 ml, MP: 80 µl/4ml, and AP: 444.7/4 ml for human serum dilution; and the antibodies had initial concentrations of 500 nM and were diluted 10 times.

### 5.2 Experimental steps

1) 50 µL of the diluted sample and 50 µL of serum were added into a plate, followed by incubation at 37°C for 1 hour; 2) the plate was washed three times with a washing buffer; 3) 100 µL of conjugate was added to each well, and the plate was placed at room temperature for 30 minutes; 4) the plate was washed three times with the washing buffer; 5) 100 µL of substrate solution was added to each well, and the plate was placed at room temperature for 30 minutes; 6) 100 µL of 5 mM EDTA was added to each well to terminate color development; and 7) an absorbance value at 450 nm was read with a multi-spectral enzyme-linked reader (ELISA) after the color development was terminated.

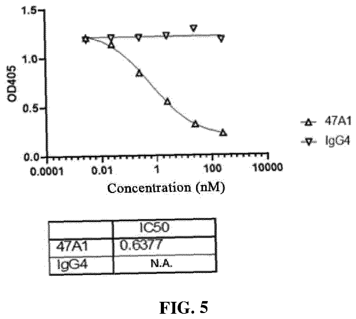

5.3 Data processing: data were processed with GraphPad Prism software. As shown in FIG. 5, the antibodies of the present application can block the lectin pathway of the human complement system.

### Example 6 Measurement on affinity of anti-MASP-2 antibody

### 6.1 Sample dilution

Affinities of candidate antibodies JYB1931A63 and JYB1931A13 to human hMASP2-D456 (KACTUS, batch number: 080203), cynomolgus monkey cMASP2-D456 (KACTUS, batch number: 030301), and mouse mMASP2-D456 (KACTUS, batch number: 030401) were measured by Octet RED96e (Fortebio). The antigen and antibody were diluted with 1xPBST (1xPBS: Sangon, B548117-0500; 0.02% Tween 20: sigma-aldrich, P1379) at a concentration of 30 nM for the antigen and a concentration of 5 µg/mL for the antibody.

### 6.2 Sample on the machine test (Octet data acquisition 11.1.0.11)

First, the sample was added to a 96-well plate (Greiner bio-one, 655209) with a system of 200 µL/well. Then, software parameters were set, the temperature of the plate was set to 30°C, and the frequency for collecting standard dynamic signals was 5.0 Hz. Next, an AHC sensor (Fortébio, article number: 18-0015) was pre-wet with 1×PBST for 10 minutes, and then data were acquired. Each cycle included the following steps: 1) immersing the sensor in a buffer solution for 60 seconds; 2) detecting whether the antigen had non-specific binding with the sensor; 3) regenerating the sensor with 10 mM pH 1.7 glycine solution; 4) immersing the sensor in the buffer solution for 60 seconds; 5) immobilizing the antibody on the sensor for 60 seconds; 6) immersing the sensor in the buffer solution for 180 seconds; 7) binding the antigen and the antibody for 180 seconds; 8) dissociating the antigen and the antibody for 5 minutes; and 9) regenerating the sensor.

### 6.3 Data analysis

A binding rate (Ka) and a dissociation rate (Kd) in a 1:1 antigen-antibody binding manner were measured by Fortebio's Data Analysis 12.0 software to calculate an equilibrium dissociation constant (K_{D}) of the antibody. The results were shown in Table 1.

**Table 1 Affinities of antibody to human hMASP2-D456, cynomolgus monkey cMASP2-D456, and mouse mMASP2-D456**

| Sample | hMASP2-D456 | cMASP2-D456 | mMASP2-D456 |
|---|---|---|---|
| | *K_{D} (M)* | *K_{D} (M)* | *K_{D} (M)* |
| JYB1931A63 | 3.35E-10 | 1.31E-09 | Unbound |
| JYB1931A13 | 1.17E-9 | 3.05E-10 | Unbound |

### Example 7: Anti-MASP-2 humanized antibodies blocked the lectin pathway of the complement system

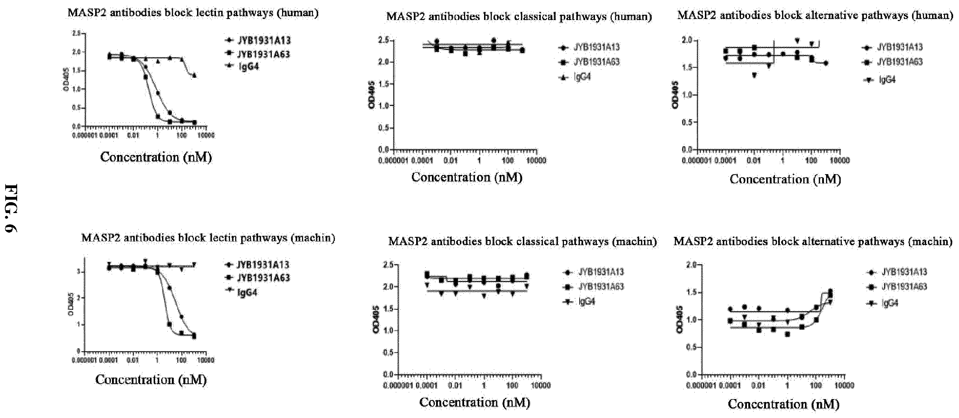

Blocking of complement signaling pathways of human serum and monkey serum by the antibodies was detected by a WIESLAB^{®} complement system lectin pathway (Svar Life Science AB, article number: AS 1327) kit. The human serum and the monkey serum were diluted with three diluents: CP: 80 µl/4 ml, MP: 80 µl/4 ml, and AP: 444.7/4 ml for human serum dilution; and CP: 2/100, MP: 2/100, and AP: 11.1/100 for monkey serum dilution. The samples were diluted with three different diluents: starting from 500 nM and diluting 10 times. The experimental steps and data processing were the same as Example 5. As shown in FIG. 6, the humanized antibodies of the present application can block the lectin pathway of the complement system.

### Example 8 Detection on physical and chemical properties of antibody

### 8.1 SEC-HPLC purity analysis

(1) The sample was diluted to 1 mg/mL, mixed well, and centrifuged at 12000 rpm for 5 minutes, the supernatant was transferred to a sample bottle, and the sample bottle was placed in an HPLC sample tray. Chromatographic conditions were set as shown in Table 2.

**Table 2 Chromatographic conditions**

| Chromatographic conditions | Parameters |
|---|---|
| Chromatographic column | TSK G3000SWxl |
| Detection wavelength | 280 nm |
| Column temperature | 25°C |
| Sample room temperature | 5°C |
| Flow rate | 0.5 ml/min |

(2) The chromatographic column was equilibrated with a mobile phase (200 mM phosphate buffer, pH 6.8), followed by injection analysis. Data were analyzed by chromatography software, and a peak area percentage of each peak was calculated by a peak area normalization method.

### 8.2 HIC-HPLC analysis

(1) The sample was diluted to 1 mg/ml and centrifuged to obtain a supernatant to be tested. Chromatographic conditions were set as follows (Table 3):

**Table 3 Chromatographic conditions**

| Chromatographic conditions | Parameters |
|---|---|
| Chromatographic column | MAbPac^{™}HIC-10 |
| Detection wavelength | 214 nm |
| Column temperature | 30°C |
| Sample room temperature | 5°C |
| Flow rate | 0.8 ml/min |

(2) Gradient elution was elutedwith mobile phase A (50 mM phosphate buffer/1 M ammonium sulfate, pH 7.0) and mobile phase B (50 mM phosphate buffer, pH 7.0), and retention time of a main peak was recorded.

### 8.3 Analysis on melting temperature (Tm) value

The test sample was diluted with a sample buffer to 1 mg/mL. Then follow instructions of Protein Thermal Shift^{™} Starter Kit: 13 µL of the test sample solution was added to a PCR tube, 5 µL of Protein Thermal shift ^{™} Buffer and 2 µL of 10 × staining solution were added to a reaction volume of 20 µL, and the solution was mixed well and centrifuged at 12000 rpm for 5 minutes to remove bubbles. The test sample was placed in a PCR instrument for sample analysis, and the Tm value of the sample was recorded.

### 8.4 iCIEF analysis

The sample solution was added to the following well-mixed system: 1% methyl cellulose (MC) 70 µl, urea 5M 80 µl, Pharmalyte pH 3-10 8 µl, and pI markers 5.5 and 9.5 1 µl each. An appropriate volume of ultra-pure water was added to 200 µl, followed by well mixing. The solution was centrifuged to obtain a supernatant for injection analysis. After the analysis was completed, the result file was imported into ChromPerfect software for graph integration processing, and an isoelectric point and percentage of each peak were calculated, as shown in Table 4.

**Table 4 Physical and chemical analysis results**

| Name of protein | Physical and chemical properties | | | | | | |
|---|---|---|---|---|---|---|---|
| | SEC% | Tm°C | HIC (min) | Measured pI | Acid peaks (%) | Main peak (%) | Basic peaks (%) |
| JYB1931A63 | 99.0 | / | 16.0 | 6.9 | 13.5 | 65.1 | 21.4 |
| JYB1931A13 | 99.5 | 82.7 | 15.8 | 9.0 | 24.3 | 58.0 | 17.6 |

### Example 9: Study on pharmacokinetics (PK) of antibody molecules in humanized FcRn mouse model

Humanized FcRn mice were used as test animals, and pharmacokinetic indicators of two to-be-tested drug positive controls (Narsoplimab) and JYB1931A63 after a single intraperitoneal administration were studied respectively. All animal experimental schemes have been reviewed and approved by IACUC. hFcRn mice were purchased from Beijing Biocytogen, male, 6-8 weeks old, weighing 23-26 g, raised in SPF grade animal rooms, fed with standard pellet feed, freely eating and drinking water, at room temperature of 18-24°C, relative humidity of 40-50%, alternating day and night for 12 hours a day. A total of 16 experimental animals were randomly divided into four groups, with four animals in each group. The animals were administered intraperitoneally in a single dose of 10 mg/kg and a volume of 10 mL/kg. Blood was collected before administration and 2 hours, 6 hours, 24 hours (day 1), day 2, day 3, day 4, day 7, day 10, day 14, day 21, day 28, day 35, and day 42 after administration. 60 µL of whole blood was collected from each animal through the orbit, stood at room temperature for 30 minutes, and then centrifuged (2000 g, 4°C, 5 minutes) to separate serum. Each sample was dispensed in 2 portions (detection tube and backup tube), 10 µL/tube, stored at -80°C.

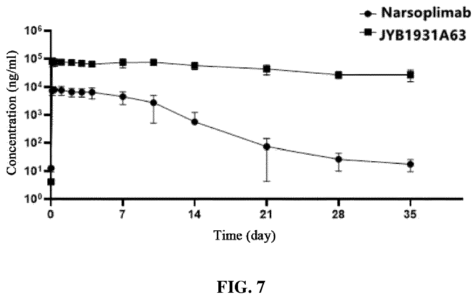

Pharmacokinetics of the four drugs at different time were analyzed by Elisa indirect detection. The coating antigen was a recombinant human MASP-2 protein, 1 µg/mL, 100 µL/well, 4°C, overnight. After the plate was washed, the sample was blocked with 200 µL/well blocking solution at 4°C overnight. The serum sample was added, 50 µL/well, 37°C, 1 hour. The detection antibody was mouse monoclonal antibody [H2] anti-human IgG F(ab)'2(HRP) (abcam, ab87422, GR3246767-11) + Streptavidin-peroxidase (Sigma, lot: SLCB5784), 100 µL/well, 37°C, 0.5 hour. TMB developing solution (KPL, article number: 52-00-03) was used for color development, and a value at OD450 was read with an ELIASA (Molecular Devices, SpectraMax M3). Drug concentrations were obtained according to a standard curve, and data were processed in a PK Solver non-compartment to obtain PK parameters. Plasma concentration curves were shown in FIG. 7.

After a positive control was administered, the half-life (t_{1/2}) was 4.93 days, the peak time (Tmax) was day 0.45, and the maximum concentration (Cmax) was 9393 ng/ml. After JYB1931A63 was administered, the half-life (t_{1/2}) was 18.6 days, the peak time (Tmax) was day 4.53, and the maximum concentration (Cmax) was 86934 ng/ml.

The foregoing detailed description is provided in a form of explanation and examples, and is not intended to limit the scope of the appended claims. At present, various changes in the implementations listed in the present application are obvious to those of ordinary skill in the art, and are retained within the scope of the appended claims and equivalent solutions thereof.

## Claims

1. An isolated antigen binding protein, having one or more of the following properties:
a) in Octet detection, specifically binding to a human MASP-2 protein at a *K_{D}* value of about 2E-09M or less;
b) in Octet detection, specifically binding to a cynomolgus monkey MASP-2 protein at a *K_{D}* value of about 2E-09M or less; and
c) capable of specifically blocking a lectin pathway of a human complement system without affecting a classical pathway and an alternative pathway of a complement.

2. The isolated antigen binding protein according to claim 1, comprising HCDR3, the HCDR3 comprising an amino acid sequence shown in SEQ ID NO: 19.

3. The isolated antigen binding protein according to any one of claims 1 and 2, comprising HCDR2, the HCDR2 comprising an amino acid sequence shown in SEQ ID NO: 17.

4. The isolated antigen binding protein according to any one of claims 1-3, comprising HCDR1, the HCDR1 comprising an amino acid sequence shown in SEQ ID NO: 15.

5. The isolated antigen binding protein according to any one of claims 1-4, comprising HCDR1, HCDR2, and HCDR3 of a heavy chain variable region VH shown in SEQ ID NO: 67.

6. The isolated antigen binding protein according to any one of claims 1-5, comprising HCDR1, HCDR2, and HCDR3 of a heavy chain variable region VH shown in any of SEQ ID NO: 13 and SEQ ID NO: 26.

7. The isolated antigen binding protein according to any one of claims 1-6, comprising a heavy chain variable region VH, the VH comprising HCDR1, HCDR2, and HCDR3, wherein the HCDR3 comprises an amino acid sequence shown in SEQ ID NO: 19; the HCDR2 comprises an amino acid sequence shown in SEQ ID NO: 17; and the HCDR1 comprises an amino acid sequence shown in SEQ ID NO: 15.

8. The isolated antigen binding protein according to any one of claims 1-7, comprising H-FR1, a C terminal of the H-FR1 isdirectly or indirectly connected to an N terminal of the HCDR1, and the H-FR1 comprising an amino acid sequence shown in SEQ ID NO: 62.

9. The isolated antigen binding protein according to claim 8, wherein the H-FR1 comprises an amino acid sequence shown in any of SEQ ID NO: 14 and SEQ ID NO: 27.

10. The isolated antigen binding protein according to any one of claims 1-9, comprising H-FR2, the H-FR2 is located between the HCDR1 and the HCDR2, and the H-FR2 comprising an amino acid sequence shown in SEQ ID NO: 63.

11. The isolated antigen binding protein according to claim 10, wherein the H-FR2 comprises an amino acid sequence shown in any of SEQ ID NO: 16 and SEQ ID NO: 28.

12. The isolated antigen binding protein according to any one of claims 1-11, comprising H-FR3, the H-FR3 is located between the HCDR2 and the HCDR3, and the H-FR3 comprising an amino acid sequence shown in SEQ ID NO: 64.

13. The isolated antigen binding protein according to claim 12, wherein the H-FR3 comprises an amino acid sequence shown in any of SEQ ID NO: 18 and SEQ ID NO: 29.

14. The isolated antigen binding protein according to any one of claims 1-13, comprising H-FR4, an N terminal of the H-FR4 is directly or indirectly connected to a C terminal of the HCDR3, and the H-FR4 comprising an amino acid sequence shown in SEQ ID NO: 65.

15. The isolated antigen binding protein according to claim 14, wherein the H-FR4 comprises an amino acid sequence shown in any of SEQ ID NO: 20 and SEQ ID NO: 30.

16. The isolated antigen binding protein according to any one of claims 1-15, comprising H-FR1, H-FR2, H-FR3, and H-FR4, wherein the H-FR1 comprises an amino acid sequence shown in SEQ ID NO: 62; the H-FR2 comprises an amino acid sequence shown in SEQ ID NO: 63; the H-FR3 comprises an amino acid sequence shown in SEQ ID NO: 64; and the H-FR4 comprises an amino acid sequence shown in SEQ ID NO: 65.

17. The isolated antigen binding protein according to claim 16, wherein the H-FR1 comprises an amino acid sequence shown in any of SEQ ID NO: 14 and SEQ ID NO: 27; the H-FR2 comprises an amino acid sequence shown in any of SEQ ID NO: 16 and SEQ ID NO: 28; the H-FR3 comprises an amino acid sequence shown in any of SEQ ID NO: 18 and SEQ ID NO: 29; and the H-FR4 comprises an amino acid sequence shown in any of SEQ ID NO: 20 and SEQ ID NO: 30.

18. The isolated antigen binding protein according to any one of claims 16 and 17, wherein the H-FR1, H-FR2, H-FR3, and H-FR4 comprise amino acid sequences selected from any of the following groups:
a) H-FR1: SEQ ID NO: 14, H-FR2: SEQ ID NO: 16, H-FR3: SEQ ID NO: 18, and H-FR4: SEQ ID NO: 20; and
b) H-FR1: SEQ ID NO: 27, H-FR2: SEQ ID NO: 28, H-FR3: SEQ ID NO: 29, and H-FR4: SEQ ID NO: 30.

19. The isolated antigen binding protein according to any one of claims 1-18, comprising a heavy chain variable region VH, the VH comprising an amino acid sequence shown in SEQ ID NO: 67.

20. The isolated antigen binding protein according to claim 19, wherein the VH comprises an amino acid sequence shown in any of SEQ ID NO: 13 and SEQ ID NO: 26.

21. The isolated antigen binding protein according to any one of claims 1-20, comprising LCDR3, the LCDR3 comprising an amino acid sequence shown in SEQ ID NO: 11.

22. The isolated antigen binding protein according to any one of claims 1-21, comprising LCDR2, the LCDR2 comprising an amino acid sequence shown in SEQ ID NO: 9.

23. The isolated antigen binding protein according to any one of claims 1-22, comprising LCDR1, the LCDR1 comprising an amino acid sequence shown in SEQ ID NO: 7.

24. The isolated antigen binding protein according to any one of claims 1-23, comprising LCDR1, LCDR2, and LCDR3 of a light chain variable region VL shown in SEQ ID NO: 66.

25. The isolated antigen binding protein according to any one of claims 1-24, comprising LCDR1, LCDR2, and LCDR3 of a light chain variable region VL shown in any of SEQ ID NO: 5 and 21.

26. The isolated antigen binding protein according to any one of claims 1-25, comprising a light chain variable region VL, the VL comprising LCDR1, LCDR2, and LCDR3, wherein the LCDR3 comprises an amino acid sequence shown in SEQ ID NO: 11; the LCDR2 comprises an amino acid sequence shown in SEQ ID NO: 9; and the LCDR1 comprises an amino acid sequence shown in SEQ ID NO: 7.

27. The isolated antigen binding protein according to any one of claims 1-26, comprising L-FR1, a C terminal of the L-FR1 is directly or indirectly connected to an N terminal of the LCDR1, and the L-FR1 comprising an amino acid sequence shown in SEQ ID NO: 58.

28. The isolated antigen binding protein according to claim 27, wherein the L-FR1 comprises an amino acid sequence shown in any of SEQ ID NO: 6 and SEQ ID NO: 22.

29. The isolated antigen binding protein according to any one of claims 1-28, comprising L-FR2, the L-FR2 is located between the LCDR1 and the LCDR2, and the L-FR2 comprising an amino acid sequence shown in SEQ ID NO: 59.

30. The isolated antigen binding protein according to claim 29, wherein the L-FR2 comprises an amino acid sequence shown in any of SEQ ID NO: 8 and SEQ ID NO: 23.

31. The isolated antigen binding protein according to any one of claims 1-30, comprising L-FR3, the L-FR3 is located between the LCDR2 and the LCDR3, and the L-FR3 comprising an amino acid sequence shown in SEQ ID NO: 60.

32. The isolated antigen binding protein according to claim 31, wherein the L-FR3 comprises an amino acid sequence shown in any of SEQ ID NO: 10 and SEQ ID NO: 24.

33. The isolated antigen binding protein according to any one of claims 1-32, comprising L-FR4, an N terminal of the L-FR4 is directly or indirectly connected to a C terminal of the LCDR3, and the L-FR4 comprising an amino acid sequence shown in SEQ ID NO: 61.

34. The isolated antigen binding protein according to claim 33, wherein the L-FR4 comprises an amino acid sequence shown in any of SEQ ID NO: 12 and SEQ ID NO: 25.

35. The isolated antigen binding protein according to any one of claims 1-34, comprising L-FR1, L-FR2, L-FR3, and L-FR4, wherein the L-FR1 comprises an amino acid sequence shown in SEQ ID NO: 58; the L-FR2 comprises an amino acid sequence shown in SEQ ID NO: 59; the L-FR3 comprises an amino acid sequence shown in SEQ ID NO: 60; and the L-FR4 comprises an amino acid sequence shown in SEQ ID NO: 61.

36. The isolated antigen binding protein according to claim 35, wherein the L-FR1 comprises an amino acid sequence shown in any of SEQ ID NO: 6 and SEQ ID NO: 22; the L-FR2 comprises an amino acid sequence shown in any of SEQ ID NO: 8 and SEQ ID NO: 23; the L-FR3 comprises an amino acid sequence shown in any of SEQ ID NO: 10 and SEQ ID NO: 24; and the L-FR4 comprises an amino acid sequence shown in any of SEQ ID NO: 12 and SEQ ID NO: 25.

37. The isolated antigen binding protein according to any one of claims 35 and 36, wherein the L-FR1, L-FR2, L-FR3, and L-FR4 comprise amino acid sequences selected from any of the following groups:
a) L-FR1: SEQ ID NO: 6, L-FR2: SEQ ID NO: 8, L-FR3: SEQ ID NO: 10, and L-FR4: SEQ ID NO: 12; and
b) L-FR1: SEQ ID NO: 22, L-FR2: SEQ ID NO: 23, L-FR3: SEQ ID NO: 24, and L-FR4: SEQ ID NO: 25.

38. The isolated antigen binding protein according to any one of claims 1-37, wherein the VL comprises an amino acid sequence shown in SEQ ID NO: 66.

39. The isolated antigen binding protein according to claim 38, wherein the VL comprises an amino acid sequence shown in any of SEQ ID NO: 5 and SEQ ID NO: 21.

40. The isolated antigen binding protein according to any one of claims 1-39, comprising a VH and a VL, wherein the VH and the VL comprise amino acid sequences selected from any of the following groups:
a) VH: SEQ ID NO: 13 and VL: SEQ ID NO: 5; and
b) VH: SEQ ID NO: 26 and VL: SEQ ID NO: 21.

41. The isolated antigen binding protein according to claim 1, comprising HCDR3, the HCDR3 comprising an amino acid sequence shown in SEQ ID NO: 45.

42. The isolated antigen binding protein according to any one of claims 1 and 41, comprising HCDR2, the HCDR2 comprising an amino acid sequence shown in SEQ ID NO: 43.

43. The isolated antigen binding protein according to any one of claims 1, 41, and 42, comprising HCDR1, the HCDR1 comprising an amino acid sequence shown in SEQ ID NO: 41.

44. The isolated antigen binding protein according to any one of claims 1 and 41-43, comprising HCDR1, HCDR2, and HCDR3 of a heavy chain variable region VH shown in SEQ ID NO: 77.

45. The isolated antigen binding protein according to any one of claims 1 and 41-44, comprising HCDR1, HCDR2, and HCDR3 of heavy chain variable regions VH shown in SEQ ID NO: 39 and SEQ ID NO: 52.

46. The isolated antigen binding protein according to any one of claims 1 and 41-45, comprising a heavy chain variable region VH, the VH comprising HCDR1, HCDR2, and HCDR3, wherein the HCDR3 comprises an amino acid sequence shown in SEQ ID NO: 45; the HCDR2 comprises an amino acid sequence shown in SEQ ID NO: 43; and the HCDR1 comprises an amino acid sequence shown in SEQ ID NO: 41.

47. The isolated antigen binding protein according to any one of claims 1 and 41-46, comprising H-FR1, a C terminal of the H-FR1 is directly or indirectly connected to an N terminal of the HCDR1, and the H-FR1 comprising an amino acid sequence shown in SEQ ID NO: 72.

48. The isolated antigen binding protein according to claim 47, wherein the H-FR1 comprises an amino acid sequence shown in any of SEQ ID NO: 40 and SEQ ID NO: 53.

49. The isolated antigen binding protein according to any one of claims 1 and 41-48, comprising H-FR2, the H-FR2 is located between the HCDR1 and the HCDR2, and the H-FR2 comprising an amino acid sequence shown in SEQ ID NO: 73.

50. The isolated antigen binding protein according to claim 49, wherein the H-FR2 comprises an amino acid sequence shown in any of SEQ ID NO: 42 and SEQ ID NO: 54.

51. The isolated antigen binding protein according to any one of claims 1 and 41-50, comprising H-FR3, the H-FR3 is located between the HCDR2 and the HCDR3, and the H-FR3 comprising an amino acid sequence shown in SEQ ID NO: 74.

52. The isolated antigen binding protein according to claim 51, wherein the H-FR3 comprises an amino acid sequence shown in any of SEQ ID NO: 44 and SEQ ID NO: 55.

53. The isolated antigen binding protein according to any one of claims 1 and 41-52, comprising H-FR4, an N terminal of the H-FR4 is directly or indirectly connected to a C terminal of the HCDR3, and the H-FR4 comprising an amino acid sequence shown in SEQ ID NO: 75.

54. The isolated antigen binding protein according to claim 53, wherein the H-FR4 comprises an amino acid sequence shown in any of SEQ ID NO: 46 and SEQ ID NO: 30.

55. The isolated antigen binding protein according to any one of claims 1 and 41-54, comprising H-FR1, H-FR2, H-FR3, and H-FR4, wherein the H-FR1 comprises an amino acid sequence shown in SEQ ID NO: 72; the H-FR2 comprises an amino acid sequence shown in SEQ ID NO: 73; the H-FR3 comprises an amino acid sequence shown in SEQ ID NO: 74; and the H-FR4 comprises an amino acid sequence shown in SEQ ID NO: 75.

56. The isolated antigen binding protein according to claim 55, wherein the H-FR1 comprises an amino acid sequence shown in any of SEQ ID NO: 40 and SEQ ID NO: 53; the H-FR2 comprises an amino acid sequence shown in any of SEQ ID NO: 42 and SEQ ID NO: 54; the H-FR3 comprises an amino acid sequence shown in any of SEQ ID NO: 44 and SEQ ID NO: 55; and the H-FR4 comprises an amino acid sequence shown in any of SEQ ID NO: 46 and SEQ ID NO: 30.

57. The isolated antigen binding protein according to any one of claims 55 and 56, wherein the H-FR1, H-FR2, H-FR3, and H-FR4 comprise amino acid sequences selected from any of the following groups:
a) H-FR1: SEQ ID NO: 40, H-FR2: SEQ ID NO: 42, H-FR3: SEQ ID NO: 44, and H-FR4: SEQ ID NO: 46; and
b) H-FR1: SEQ ID NO: 53, H-FR2: SEQ ID NO: 54, H-FR3: SEQ ID NO: 55, and H-FR4: SEQ ID NO: 30.

58. The isolated antigen binding protein according to any one of claims 1 and 41-57, comprising a heavy chain variable region VH, the VH comprising an amino acid sequence shown in SEQ ID NO: 77.

59. The isolated antigen binding protein according to claim 58, wherein the VH comprises an amino acid sequence shown in any of SEQ ID NO: 39 and SEQ ID NO: 52.

60. The isolated antigen binding protein according to any one of claims 1 and 41-59, comprising LCDR3, the LCDR3 comprising an amino acid sequence shown in SEQ ID NO: 37.

61. The isolated antigen binding protein according to any one of claims 1 and 41-60, comprising LCDR2, the LCDR2 comprising an amino acid sequence shown in SEQ ID NO: 35.

62. The isolated antigen binding protein according to any one of claims 1 and 41-61, comprising LCDR1, the LCDR1 comprising an amino acid sequence shown in SEQ ID NO: 33.

63. The isolated antigen binding protein according to any one of claims 1 and 41-62, comprising LCDR1, LCDR2, and LCDR3 of a light chain variable region VL shown in SEQ ID NO: 76.

64. The isolated antigen binding protein according to any one of claims 1 and 41-63, comprising LCDR1, LCDR2, and LCDR3 of light chain variable regions VL shown in SEQ ID NO: 31 and SEQ ID NO: 47.

65. The isolated antigen binding protein according to any one of claims 1 and 41-64, comprising a light chain variable region VL, the VL comprising LCDR1, LCDR2, and LCDR3, wherein the LCDR3 comprises an amino acid sequence shown in SEQ ID NO: 37; the LCDR2 comprises an amino acid sequence shown in SEQ ID NO: 35; and the LCDR1 comprises an amino acid sequence shown in SEQ ID NO: 33.

66. The isolated antigen binding protein according to any one of claims 1 and 41-65, comprising L-FR1, a C terminal of the L-FR1 is directly or indirectly connected to an N terminal of the LCDR1, and the L-FR1 comprising an amino acid sequence shown in SEQ ID NO: 68.

67. The isolated antigen binding protein according to claim 66, wherein the L-FR1 comprises an amino acid sequence shown in any of SEQ ID NO: 32 and SEQ ID NO: 48.

68. The isolated antigen binding protein according to any one of claims 1 and 41-67, comprising L-FR2, the L-FR2 is located between the LCDR1 and the LCDR2, and the L-FR2 comprising an amino acid sequence shown in SEQ ID NO: 69.

69. The isolated antigen binding protein according to claim 68, wherein the L-FR2 comprises an amino acid sequence shown in any of SEQ ID NO: 34 and SEQ ID NO: 49.

70. The isolated antigen binding protein according to any one of claims 1 and 41-69, comprising L-FR3, the L-FR3 being located between the LCDR2 and the LCDR3, and the L-FR3 comprising an amino acid sequence shown in SEQ ID NO: 70.

71. The isolated antigen binding protein according to claim 70, wherein the L-FR3 comprises an amino acid sequence shown in any of SEQ ID NO: 36 and SEQ ID NO: 50.

72. The isolated antigen binding protein according to any one of claims 1 and 41-71, comprising L-FR4, an N terminal of the L-FR4 is directly or indirectly connected to a C terminal of the LCDR3, and the L-FR4 comprising an amino acid sequence shown in SEQ ID NO: 71.

73. The isolated antigen binding protein according to claim 72, wherein the L-FR4 comprises an amino acid sequence shown in any of SEQ ID NO: 38 and SEQ ID NO: 51.

74. The isolated antigen binding protein according to any one of claims 1 and 41-73, comprising L-FR1, L-FR2, L-FR3, and L-FR4, wherein the L-FR1 comprises an amino acid sequence shown in SEQ ID NO: 68; the L-FR2 comprises an amino acid sequence shown in SEQ ID NO: 69; the L-FR3 comprises an amino acid sequence shown in SEQ ID NO: 70; and the L-FR4 comprises an amino acid sequence shown in SEQ ID NO: 71.

75. The isolated antigen binding protein according to claim 74, wherein the L-FR1 comprises an amino acid sequence shown in any of SEQ ID NO: 32 and SEQ ID NO: 48; the L-FR2 comprises an amino acid sequence shown in any of SEQ ID NO: 34 and SEQ ID NO: 49; the L-FR3 comprises an amino acid sequence shown in any of SEQ ID NO: 36 and SEQ ID NO: 50; and the L-FR4 comprises an amino acid sequence shown in any of SEQ ID NO: 38 and SEQ ID NO: 51.

76. The isolated antigen binding protein according to any one of claims 74 and 75, wherein the L-FR1, L-FR2, L-FR3, and L-FR4 comprise amino acid sequences selected from any of the following groups:
a) L-FR1: SEQ ID NO: 32, L-FR2: SEQ ID NO: 34, L-FR3: SEQ ID NO: 36, and L-FR4: SEQ ID NO: 38; and
b) L-FR1: SEQ ID NO: 48, L-FR2: SEQ ID NO: 49, L-FR3: SEQ ID NO: 50, and L-FR4: SEQ ID NO: 51.

77. The isolated antigen binding protein according to any one of claims 1 and 41-76, comprising a VL, the VL comprising an amino acid sequence shown in SEQ ID NO: 76.

78. The isolated antigen binding protein according to claim 77, wherein the VL comprises an amino acid sequence shown in any of SEQ ID NO: 31 and 47.

79. The isolated antigen binding protein according to any one of claims 1 and 41-78, comprising a VH and a VL, wherein the VH and the VL comprise amino acid sequences selected from any of the following groups:
a)VH: SEQ ID NO: 39 and VL: SEQ ID NO: 31; and
b) VH: SEQ ID NO: 52 and VL: SEQ ID NO: 47.

80. The isolated antigen binding protein according to any one of claims 1-79, comprising a heavy chain constant region, and the heavy chain constant region comprising a constant region derived from IgG or a constant region derived from IgY.

81. The isolated antibody binding protein according to claim 80, wherein the heavy chain constant region comprises a constant region derived from IgG.

82. The isolated antigen binding protein according to any one of claims 80 and 81, wherein the heavy chain constant region comprises a constant region derived from IgG1, IgG2, IgG3, or IgG4.

83. The isolated antigen binding protein according to any one of claims 80-82, wherein the heavy chain constant region comprises an amino acid sequence shown in SEQ ID NO: 56.

84. The isolated antigen binding protein according to any one of claims 1-83, comprising a light chain constant region, and the light chain constant region comprising a constant region derived from Igκ or a constant region derived from Igλ.

85. The isolated antigen binding protein according to claim 84, wherein the light chain constant region comprises a constant region derived from human Igκ.

86. The isolated antigen binding protein according to any one of claims 84 and 85, wherein the light chain constant region comprises an amino acid sequence shown in SEQ ID NO: 57.

87. The isolated antigen binding protein according to any one of claims 1-86, comprising an antibody or an antigen binding fragment thereof.

88. The isolated antigen binding protein according to claim 87, wherein the antigen binding fragment is selected from the following group: Fab, Fab', F(ab)2, Fv fragment, F(ab')2, scFv, di-scFv, VHH, and/or dAb.

89. The isolated antigen binding protein according to any one of claims 87 and 88, wherein the antibody is selected from the following group: a monoclonal antibody, a single chain antibody, a chimeric antibody, a humanized antibody, and a fully human antibody.

90. A polypeptide, comprising the isolated antigen binding protein according to any one of claims 1-89.

91. An immunoconjugate, comprising the isolated antigen binding protein according to any one of claims 1-89 or the polypeptide according to claim 90.

92. An isolated nucleic acid molecule, encoding the isolated antigen binding protein according to any one of claims 1-89 or the polypeptide according to claim 90.

93. A vector, comprising the isolated nucleic acid molecule according to claim 92.

94. A cell, comprising the isolated antigen binding protein according to any one of claims 1-89, the polypeptide according to claim 90, the immunoconjugate according to claim 91, the isolated nucleic acid molecule according to claim 92, and/or the vector according to claim 93.

95. A method for preparing the isolated antigen binding protein according to any one of claims 1-89 or the polypeptide according to claim 90, the method comprising culturing the cell according to claim 94 under the condition of expressing the isolated antigen binding protein according to any one of claims 1-89 or the polypeptide according to claim 90.

96. A pharmaceutical composition, comprising the isolated antigen binding protein according to any one of claims 1-89, the polypeptide according to claim 90, the immunoconjugate according to claim 91, the isolated nucleic acid molecule according to claim 92, the vector according to claim 93, the cell according to claim 94, and/or pharmaceutically acceptable adjuvants and/or excipients.

97. A method for detecting or measuring MASP-2, comprising use of the isolated antigen binding protein according to any one of claims 1-89 or the polypeptide according to claim 90.

98. A detection kit for MASP-2, comprising the isolated antigen binding protein according to any one of claims 1-89 or the polypeptide according to claim 90.

99. A use of the isolated antigen binding protein according to any one of claims 1-89 or the polypeptide according to claim 90 in preparation of a kit for detecting the presence and/or content of MASP-2.

100. A use of the isolated antigen binding protein according to any one of claims 1-89, the polypeptide according to claim 90, the immunoconjugate according to claim 91, the isolated nucleic acid molecule according to claim 92, the vector according to claim 93, the cell according to claim 94, and/or the pharmaceutical composition according to claim 96 in preparation of drugs for preventing and/or treating diseases or conditions.

101. A use of the isolated antigen binding protein according to any one of claims 1-89, the polypeptide according to claim 90, the immunoconjugate according to claim 91, the isolated nucleic acid molecule according to claim 92, the vector according to claim 93, the cell according to claim 94, and/or the pharmaceutical composition according to claim 96 for preventing, alleviating, and/or treating diseases or conditions.

102. A method for preventing and/or treating diseases or conditions, comprising administering, to subjects in need, an effective amount of the isolated antigen binding protein according to any one of claims 1-89, the polypeptide according to claim 90, the immunoconjugate according to claim 91, the isolated nucleic acid molecule according to claim 92, the vector according to claim 93, the cell according to claim 94, and/or the pharmaceutical composition according to claim 96.
